# EUROPEAN PATENT SPECIFICATION

(11) **EP 1 350 523 B1**
(45) Date of publication and mention of the grant of the patent: **16.05.2007**
(21) Application number: 03015404.1
(22) Date of filing: 25.09.1992
(51) Int. Cl.: A61K 39/00, A61K 47/48, A61K 31/4025

(54) **Therapeutic conjugates inhibiting vascular smooth muscle cells**
Therapeutische Konjugate die die vaskuläre glatten Muskelzellen inhibitieren
Conjugués thérapeutiques inhibitant les cellules des muscles lisses vasculaires

(43) Date of publication of application: 08.10.2003
(62) Divisional of application: 94911762.6
(73) Proprietor: Boston Scientific Limited, St. Michael, Barbados, West Indies (BB)
(72) Inventor: Kunz, Lawrence L., Ellensburg, WA 98926 (US)
(74) Representative: Jones Day

(56) References cited:
- EP-A- 0 124 502
- EP-A- 0 134 320
- WO-A-93/04191
- COMEZOGLU F T ET AL: "SERUM STABILITY AND CYTOTOXICITY OF THE MACROCYCLIC TRICHOTHECENES RORIDIN A VERRUCARIN A AND THEIR MONOCLONAL ANTIBODY CONJUGATES" PROCEEDINGS AMERICAN ASSOCIATION FOR CANCER RESEARCH ANNUAL MEETING, vol. 31, 1990, page 291, XP0008022622 & ISSN: 0197-016X
- VRUDHULA V M ET AL: "SELECTIVE SYNTHETIC TRANSFORMATIONS WITH RORIDIN A" ABSTRACTS OF PAPERS AMERICAN CHEMICAL SOCIETY, vol. 199, no. 1-2, 1990, page MEDI 50, XP0008022621 & ISSN: 0065-7727

## Description

### Field of the Invention

This invention relates generally to therapeutic methods involving surgical or intravenous introduction of binding partners directed to certain target cell populations such as smooth muscle proteins, cancer cells and effector cells of the immune system, particularly for treating conditions such as stenosis following vascular trauma or disease, cancer and diseases that are mediated by immune system effector cells.

US-A 4 867 973 relates to antibody-therapeutic agent conjugates having a therapeutic agent covalently attached to an antibody or antibody fragment. As potential targets of the antibodies, US-A 4 867 973 discloses antigenic determinants of tumor cells, viruses, bacteria, or parasites (see col. 13, 2^{nd} paragraph) or mycoplasma, histocompatibility, differentiation and cell membrane antigens (col. 15, 1^{st} paragraph).

### Background of the Invention

Percutaneous transluminal coronary angioplasty (PTCA) is widely used as the primary treatment modality in many patients with coronary artery disease. PTCA can relieve myocardial ischemia in patients with coronary artery disease by reducing lumen obstruction and improving coronary flow. The use of this surgical procedure has grown rapidly, with 39, 000 procedures performed in 1983, nearly 150,000 in 1987, 200,000 in 1988, 250,000 in 1989, and over 500,000 PTCAs per year are estimated by 1994 (1, 2, 3). Stenosis following PTCA remains a significant problem, with from 25% to 35% of the patients developing restenosis within 1 to 3 months. Restenosis results in significant morbidity and mortality and frequently necessitates further interventions such as repeat angioplasty or coronary bypass surgery. No surgical intervention or post-surgical treatment (to date) has proven effective in preventing restenosis.

The processes responsible for stenosis after PTCA are not completely understood but may result from a complex interplay among several different biologic agents and pathways. Viewed in histological sections, restenotic lesions may have an overgrowth of smooth muscle cells in the intimal layers of the vessel (3). Several possible mechanisms for smooth muscle cell proliferation after PTCA have been suggested (1, 2, 4, 5).

Compounds that reportedly suppress smooth muscle proliferation *in vitro* (4, 6, 7) may have undesirable pharmacological side effects when used *in vivo.* Heparin is an example of one such compound, which reportedly inhibits smooth muscle cell proliferation *in vitro* but when used *in vivo* has the potential adverse side effect of inhibiting coagulation. Heparin peptides, while having reduced anti-coagulant activity, have the undesirable pharmacological property of having a short pharmacological half-life. Attempts have been made to solve such problems by using a double balloon catheter, i.e., for regional delivery of the therapeutic agent at the angioplasty site (e.g., 8; U.S. Pat. No. 4,824,436), and by using biodegradable materials impregnated with a drug, i.e., to compensate for problems of short half-life (e.g., 9; U.S. Pat. No. 4,929,602).

Verrucarins and Roridins are trichothecene drugs produced as secondary metabolites by the soil fungi *Myrothecium verriucaria and Myrothecium roridium.* Verrucarin is a macrocyclic triester. Roridin is a macrocyclic diester of verrucarol (10). As a group, the trichothecenes are structurally related to sesquiterpenoid mycotoxins produced by several species of fungi and characterized by the 12,13-epoxytrichothec-9-ene basic structure. Their cytotoxic activity to eukaryotic cells is closely correlated with their ability to bind to the cell, to be internalized, and to inhibit protein and macromolecular synthesis in the cell.

At least five considerations would, on their face, appear to preclude use of inhibitory drugs to prevent stenosis resulting from overgrowth of smooth muscle cells. First, inhibitory agents may have systemic toxicity that could create an unacceptable level of risk for patients and with cardiovascular disease. Second, inhibitory agents might interfere with vascular wound healing following surgery and that could either delay healing or weaken the structure or elasticity of the newly healed vessel wall. Third, inhibitory agents killing smooth muscle cells could damage surrounding endothelium and/or other medial smooth muscle cells. Dead and dying cells also release mitogenic agents that might stimulate additional smooth muscle cell proliferation and exacerbate stenosis. Fourth, delivery of therapeutically effective levels of an inhibitory agent may be problematic from several standpoints: namely, a) delivery of a large number of molecules into the intercellular spaces between smooth muscle cells may be necessary, i.e., to establish favorable conditions for allowing a therapeutically effective dose of molecules to cross the cell membrane; b) directing an inhibitory drug into the proper intracellular compartment, i.e., where its action is exerted, may be difficult to control; and, c) optimizing the association of the inhibitory drug with its intracellular target, e.g, a ribosome, while minimizing intercellular redistribution of the drug, e.g. to neighboring cells, may be difficult. Fifth, because smooth muscle cell proliferation takes place over several weeks it would appear *a priori* that the inhibitory drugs should also be administered over several weeks, perhaps continuously, to produce a beneficial effect.

As is apparent from the foregoing, many problems remain to be solved in the use of inhibitory drugs, including cytotoxic agents, to effectively treat smooth muscle cell proliferation. It would be highly advantageous to develop new methods for inhibiting stenosis due to proliferation of vascular smooth muscle cells following traumatic injury to vessels such as occurs during vascular surgery. In addition, delivery of compounds that produce inhibitory effects of extended duration to the vascular smooth muscle cells would be advantageous. Local administration of such sustained release compounds would also be useful in the treatment of other conditions where the target cell population is accessible by such administration.

The invention relates to a therapeutic conjugate comprising a binding protein or peptide coupled to a therapeutic agent, wherein said binding protein or peptide specifically binds to the cell membranes of either vascular smooth muscle cells or pericytes, or which binds to the interstitial matrix of the artery wall, and wherein said therapeutic agent alters the cellular metabolism, or inhibits a cellular activity of the smooth muscle cells or pericytes.

The invention further relates to a use of said therapeutic conjugate for the preparation of a therapeutically effective dosage of a medicament for the reduction of stenosis following angioplasty.

### Summary of the Invention

In one aspect of the invention, new therapeutic methods and therapeutic conjugates are disclosed for inhibiting vascular smooth muscle cells in a mammalian host. The therapeutic conjugates contain a vascular smooth muscle binding protein or peptide that binds in a specific manner to the cell membranes of a vascular smooth muscle cell or an interstitial matrix binding protein/peptide that binds in a specific manner to interstitial matrix (e.g., collagen) of the artery wall, coupled to a therapeutic agent that inhibits the activity of the cell. In one embodiment, inhibition of cellular activity results in reducing, delaying, or eliminating stenosis after angioplasty or other vascular surgical procedures. The therapeutic conjugates of the invention achieve these advantageous effects by associating with vascular smooth muscle cells and pericytes, which may transform into smooth muscle cells. The therapeutic conjugate may contain therapeutic agents that alter cellular metabolism or are inhibitors of protein synthesis, cellular proliferation, or cell migration, or microtubule and microfilament inhibitors that affect morphology, increases in cell volume, and/or inhibitors of extracellular matrix synthesis or secretion. In one representative embodiment, the conjugates include a cytotoxic therapeutic agent that is a sesquiterpenoid mycotoxin such as a verrucarin or a roridin. Other embodiments involve cytostatic therapeutic agents that inhibit DNA synthesis and proliferation at doses that have a minimal effect op protein synthesis such as protein kinase inhibitors (e.g., staurosporin), suramin, and nitric oxide releasing compounds (e.g., nitroglycerin) or analogs or functional equivalents thereof. Other aspects of the invention relate to vascular smooth muscle binding proteins that specifically associate with a chondroitin sulfate proteoglycan (CSPG) expressed on the membranes of a vascular smooth muscle cell, and in a preferred embodiment this CSPG has a molecular weight of about 250kDaltons. In preferred

embodiments the vascular smooth muscle binding protein binds to a CSPG target on the cell surface with an association constant of at least 10⁻⁴M. In other preferred embodiment the vascular smooth muscle binding protein contains a sequence of amino acids found in the Fab, Fv or CDR (complementary determining regions) of monoclonal antibody NR-AN-01 or functional equivalents thereof.

Other aspects of the invention include methods for inhibiting stenosis, e.g., following angioplasty in a mammalian host by administering to a human or animal subject in need of such treatment a therapeutically effective dosage of a therapeutic conjugate of the invention. In one representative embodiment the dosage of therapeutic conjugate may be administered with an infusion catheter, to achieve a 10⁻³M to 10⁻¹²M concentration of said therapeutic conjugate at the site of administration in a blood vessel.

The present invention also contemplates therapeutic methods and therapeutic dosage forms involving sustained release of therapeutic agent to target cells. Preferably, the target cells are vascular smooth muscle cells, cancer cells, and cells involved in immune system-mediated diseases that are accessible by local administration of the dosage form. Consequently, the methods and dosage forms of this aspect of the present invention are useful for inhibiting vascular smooth muscle cells in a mammalian host, employing a therapeutic agent that inhibits the activity of the cell but does not kill the cell and a vascular smooth muscle cell binding protein. Also, the methods and dosage forms of this aspect of the present invention are useful for inhibiting target cell proliferation or killing such target cells, employing a therapeutic agent that inhibits proliferation or is cytotoxic to the target cells and a tumor cell binding protein. In addition, the methods and dosage forms of this aspect of the present invention are useful for delivering cytostatic, cytocidal or metabolism modulating therapeutic agents to target cells, such as effector cells of the immune system, that are accessible by local administration of the dosage form, employing a target cell binding protein. Finally, dosage forms of the present invention are useful to reduce or eliminate pathological proliferation of normal tissue.

The dosage forms of the present invention are preferably either non-degradable microparticulates or nanoparticulates or biodegradable microparticulates or nanoparticulates. More preferably, the microparticles or nanoparticles are formed of a polymer containing matrix that biodegrades by random, nonenzymatic, hydrolytic scissioning. Such a preferred structure is formed of a mixture of thermoplastic polyesters (e.g., polylactide or polyglycolide) or a copolymer of lactide and glycolide components. The lactide/glycolide structure has the added advantage that biodegradation thereof forms lactic acid and glycolic acid, both normal metabolic products of mammals.

Preferable therapeutic agents dispersed within the microparticulates or nanoparticulates are those exhibiting inhibition of a therapeutically significant target cell activity without killing the target cell or target cell killing activity. For treatment of restenosis of vascular smooth muscle cells, useful therapeutic agents inhibit target cell activity (e.g., proliferation or migration) without killing the target cells. Preferred therapeutic moieties for this purpose are protein kinase inhibitors (e.g., staurosporin), suramin, and nitric oxide releasing compounds such as nitroglycerin or analogs or functional equivalents thereof. In cancer therapy, useful therapeutic agents inhibit proliferation or are cytotoxic to the target cells. Preferred therapeutic moieties for this purpose are Roridin A and *Pseudomonas exotoxin* or analogs or functional equivalents thereof. For treatment of immune system-modulated diseases such as arthritis, useful therapeutic agents deliver cytostatic, cytocidal or metabolism modulating therapeutic agents to target cells that are accessible by local administration of the dosage form. Preferred therapeutic moieties for this purpose are Roridin A, *Pseudomonas exotoxin,* suramin and protein kinase inhibitors (e.g., staurosporin) or analogs or functional equivalents thereof. For treatment of pathologically proliferating normal tissues, antiproliferative agents are preferred.

The dosage forms of the present invention are targeted to a relevant target cell population by a binding protein or peptide. Preferred binding proteins/peptides of the present invention are vascular smooth muscle cell binding protein, tumor cell binding protein and immune system effector cell binding protein. Preferred vascular smooth muscle cell binding proteins specifically associate with a chondroitin sulfate proteoglycan (CSPG) expressed on the membranes of a vascular smooth muscle cell, and in a preferred embodiment this CSPG has a molecular weight of about 250kDaltons. In preferred embodiments, the vascular smooth muscle binding protein binds to a CSPG target on the cell surface with an association constant of at least 10⁻⁴M. In other preferred embodiments, the vascular smooth muscle binding protein contains a sequence of amino acids found in the Fab, Fv or CDR (complementary determining regions) of monoclonal antibody-NR-AN-01 or functional equivalents thereof. Other preferred binding peptides useful in this embodiment of the present invention include those that localize to intercellular stroma and matrix located between and among vascular smooth muscle cells. Preferred binding peptides of this type are specifically associated with collagen, reticulum fibers or other intercellular matrix compounds. Preferred tumor cell binding proteins are associated with surface cell markers expressed by the target tumor cell population or cytoplasmic epitopes thereof. Preferred immune system-modulated target cell binding proteins are associated with cell surface markers of the target immune system effector cells or cytoplasmic epitopes thereof. Binding peptides/proteins of the present invention also target pathologically proliferating normal tissues.

### Description of the Drawings

FIGURE 1 A, B show a photomicrograph of a vascular smooth muscle cells in an artery of a 24-year-old male patient with vascular smooth muscle binding protein bound to the cell surface and membrane. The patient received the vascular smooth muscle binding protein by i.v. administration 4 days before the arterial tissue was prepared for histology.
FIGURE 2 depicts a first scheme for chemical coupling of a therapeutic agent to a vascular smooth muscle binding protein.
FIGURE 3 depicts a second scheme for chemical coupling of a therapeutic agent to a vascular smooth muscle binding protein.
FIGURE 4A graphically depicts experimental data showing rapid binding of vascular smooth muscle binding protein to marker-positive test cells *in vitro.*
FIGURE 4B graphically depicts experimental data showing rapid binding of vascular smooth muscle binding protein to vascular smooth muscle cells *in vitro*.
FIGURE 5A presents graphically experimental data showing undesirable cytotoxicity of even low levels of therapeutic conjugate (i.e., RA-NR-AN-01), and the free RA therapeutic agent, when vascular smooth muscle cells were treated for 24 hours *in vitro.*
FIGURE 5B graphically presents experimental data showing the effects of RA-NR-AN-01 therapeutic conjugate on metabolic activity of marker-positive and -negative cells. The data shows undesirable nonspecific cytotoxicity of the conjugate for all these cells in a 24 hours treatment *in vitro.* The non-specificity results from extracellular hydrolysis of the coupling ligand which exposes the tested cells to free drug.
FIGURE 6A graphically depicts experimental data showing undesirable nonspecific cytotoxicity of PE-NR-AN-01 therapeutic conjugate for marker-positive and marker-negative test cells after 24 hours of treatment *in vitro,* even though the 24 hour treatment was followed by an overnight recovery period prior to testing the metabolic activity.
FIGURE 6B depicts experimental data showing nonspecific cytotoxicity of the free PE therapeutic agent on marker-positive and -negative test cells after 24 hours of treatment *in vitro.*
FIGURE 7A graphically presents experimental data showing that a short 5 minute "pulse" treatment, i.e., instead of 24 hours, followed by exposure to [3H]leucine, with free RA therapeutic agent being nonspecifically cytotoxic, i.e., for control HT29 marker-negative cells, but, in contrast, the RA-NR-AN-01 therapeutic conjugate is not cytotoxic in this "pulse" treatment.
- FIGURE 7B presents graphically experimental data showing that free RA therapeutic agent is nonspecifically cytotoxic for control HT29 marker-negative cells even in a 5' "pulse" treatment followed by a 24 hour recovery period prior to [3H] leucine exposure, but, in contrast, the RA-NR-AN-01 therapeutic conjugate is not cytotoxic to cells.
FIGURE 7C presents graphically results of experiments showing that "pulse" treatment of cells *in vitro* with the RA-NR-AN-01 therapeutic conjugate inhibits cellular activity in marker-positive A375 cells as measured by protein synthesis.
FIGURE 7D presents graphically experimental data showing that "pulse" treatment of cells *in vitro* with the RA-NR-AN-01 therapeutic conjugate did not exert long-lasting inhibitory effects on cellular activity in marker-positive cells, since protein synthesis in A375 cells was not inhibited when the cells were allowed an overnight recovery period prior to testing *in vitro.*
FIGURE 8A presents graphically experimental data showing that while a "pulse" treatment of cells *in vitro* with free RA therapeutic agent was non-specifically cytotoxic, the RA-NR-AN-01 therapeutic conjugate did not exert long-lasting inhibitory effects on cellular activity in vascular smooth muscle cells as evidenced by metabolic activity in BO54 cells that were allowed a 48 hour recovery period prior to testing.
FIGURE 8B graphically depicts experimental data similar to those presented in FIGURE 8A, above, but using a second marker-positive cell type, namely A375, the data show that "pulse" treatment with the RA-NR-AN-01 therapeutic conjugate did not exert long-lasting inhibitory effects on cellular activity as measured by metabolic activity in A375 cells that were allowed a 48 hour recovery period prior to testing.
FIGURE 8C graphically depicts results similar to those presented in FIGURE 8A and FIGURE 8B, above, but using a marker-negative control cell type, namely HT29. The results show that the pulse" treatment with the RA-NR-AN-01 therapeutic conjugate did not exert long-lasting inhibitory effects on the cellular activity of marker-negative control cells as measured by metabolic activity in HT29 cells that were allowed a 48 hour recovery period prior to testing.
FIGURE 9A shows stenosis due to intimal smooth muscle cell proliferation in a histological section of an untreated artery 5 weeks after angioplasty in an animal model.
FIGURE 9B shows inhibition of stenosis in a histological section of an artery treated with therapeutic conjugate at 5 weeks after angioplasty in an animal model.
FIGURE 10A graphically depicts experimental data comparing protein synthesis and DNA synthesis inhibition capability of suramin with respect to vascular smooth muscle cells.
FIGURE 10B graphically depicts experimental data comparing protein synthesis and DNA synthesis inhibition capability of staurosporin with respect to vascular smooth muscle cells.
FIGURE 10C graphically depicts experimental data comparing protein synthesis and DNA synthesis inhibition capability of nitroglycerin with respect to vascular smooth muscle cells.
FIGURE 11 shows a tangential section parallel to the inner surface of a smooth muscle cell which is magnified 62,500 times and is characterized by numerous endocytic vesicles, several of which contain antibody coated gold beads in the process of being internalized by the cell in vitro.
FIGURE 12 shows a smooth muscle cell which is magnified 62,500 times and is characterized by a marked accumulation of gold beads in lysosomes at 24 hours following exposure of the cell to the beads in vitro.

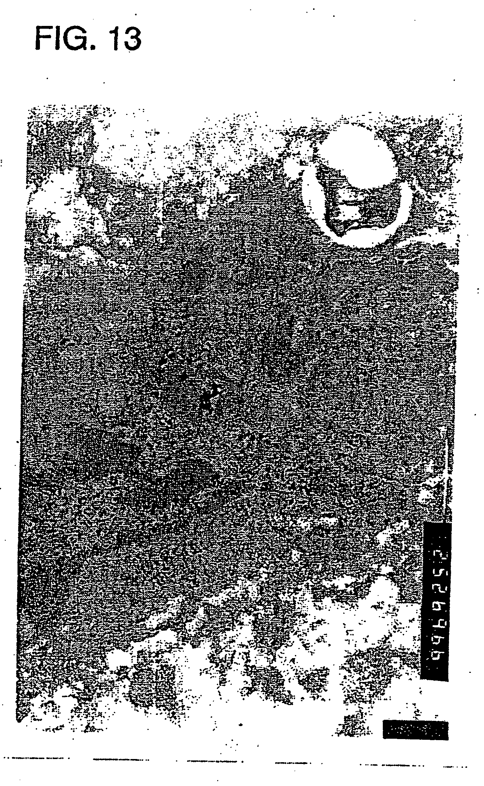

FIGURE 13 shows a smooth muscle cell which is magnified 62,500 times and is characterized by accumulation of gold beads in lysosomes in vivo.

### Detailed Description of the Invention

As used herein the following terms have the meanings as set forth below:
"Therapeutic conjugate" means a vascular smooth muscle or an interstitial matrix binding protein coupled (e.g., optionally through a linker) to a therapeutic agent.
"Targets" and "marker" are used interchangeably in describing the conjugate aspects of the present invention to mean a molecule recognized in a specific manner by the matrix or vascular smooth muscle binding protein, e.g., an antigen, polypeptide antigen or cell surface carbohydrate (e.g., a glycolipid, glycoprotein, or proteoglycan) that is expressed on the cell surface membranes of a vascular smooth muscle cell or a matrix structure.
"Epitope" is used to refer to a specific site within the "target" molecule that is bound by the matrix or smooth muscle binding protein, e.g., a sequence of three or more amino acids or saccharides.
"coupled" is used to mean covalent or non-covalent chemical association, (i.e., hydrophobic as through van der Waals forces or charge-charge interactions) of the matrix or vascular smooth muscle binding protein with the therapeutic agent. Due to the nature of the therapeutic agents employed, the binding proteins will normally be associated with the therapeutic agents by means of covalent bonding.
"Linker" means an agent that couples the matrix or smooth muscle binding protein to a therapeutic agent, e.g., an organic chemical coupler.
"Migration" of smooth muscle cells means movement of these cells in *viv*o from the medial layers of a vessel into the intima, such as may also be studied *in vitro* by following the motion of a cell from one location to another (e.g., using time-lapse cinematography or a video recorder and manual counting of smooth muscle cell migration out of a defined area in the tissue culture over time).
"Proliferation," i.e., of smooth muscle cells or cancer cells, means increase in cell number, i.e., by mitosis of the cells.
"Expressed" means mRNA transcription and translation with resultant synthesis, glycosylation, and/or secretion of a polypeptide by a cell, e.g., CSPG synthesized by a vascular smooth muscle cell or pericyte.
"Macrocyclic trichothecene" is intended to mean any one of the group of structurally related sesquiterpenoid macrocyclic mycotoxins produced by several species of fungi and characterized by the 12,13-epoxytrichothec-9-ene basic structure, e.g., verrucarins and roridins that are the products of secondary metabolism in the soil fungi *Myrothecium verriucaria* and *Myrothecium roridium*.
"Sustained release" means a dosage form designed to release a therapeutic agent therefrom for a time period ranging from about 3 to about 21 days. Release over a longer time period is also contemplated as a "sustained release" dosage form of the present invention.
"Dosage form" means a microparticulate or nanoparticulate, biodegradable or non-biodegradable polymeric material capable of binding to one or more binding proteins or peptides to deliver a therapeutic moiety dispersed therein to a target cell population.

As referred to herein, smooth muscle cells and pericytes include those cells derived from the medial layers of vessels and adventitia vessels which proliferate in intimal hyperplastic vascular sites following injury, such as that caused during PTCA. Characteristics of smooth muscle cells include a histological morphology (under light microscopic examination) of a spindle shape with an oblong nucleus located centrally in the cell with nucleoli present and myofibrils in the sarcoplasm. Under electron microscopic examination, smooth muscle cells have long slender mitochondria in the juxtanuclear sarcoplasm, a few tubular elements of granular endoplasmic reticulum, and numerous clusters of free ribosomes. A small Golgi complex may also be located near one pole of the nucleus. The majority of the sarcoplasm is occupied by thin, parallel myofilaments that may be, for the most part, oriented to the long axis of the muscle cell. These actin containing myofibrils may be arranged in bundles with mitochondria interspersed among them. Scattered through the contractile substance of the cell may also be oval dense areas, with similar dense areas distributed at intervals along the inner aspects of the plasmalemma. Characteristics of pericytes include a histological morphology (under light microscopic examination) characterized by an irregular cell shape. Pericytes are found within the basement membrane that surrounds vascular endothelial cells and their identity may be confirmed by positive immuno-staining with antibodies specific for alpha smooth muscle actin (e.g., anti-alpha-sm1, Biomakor, Rehovot, Israel), HMW-MAA, and pericyte ganglioside antigens such as MAb 3G5 (11); and, negative immune-staining with antibodies to cytokeratins (i.e., epithelial and fibroblast markers) and von Willdebrand factor (i.e., an endothelial marker).

The therapeutic conjugates and dosage forms of the invention are useful for inhibiting the activity of vascular smooth muscle cells, e.g., for reducing, delaying, or eliminating stenosis following angioplasty. As used herein the term "reducing" means decreasing the intimal thickening that results from stimulation of smooth muscle cell proliferation following angioplasty, either in an animal model or in man. "Delaying" means delaying the time until onset of visible intimal hyperplasia (e.g., observed histologically or by angiographic examination) following angioplasty and may also be accompanied by "reduced" restenosis. "Eliminating" restenosis following angioplasty means completely "reducing" and/or completely "delaying" intimal hyperplasia in a patient to an extent which makes it no longer necessary to surgically intervene, i.e., to re-establish a suitable blood flow through the vessel by repeat angioplasty, arthrectomy, or coronary artery bypass surgery. The effects of reducing, delaying, or eliminating stenosis may be determined by methods routine to those skilled in the art including, but not limited to, angiography, ultrasonic evaluation, fluoroscopic imaging, fiber optic endoscopic examination or biopsy and histology. The therapeutic conjugates of the invention achieve these advantageous effects by specifically binding to the cellular membranes of smooth muscle cells and pericytes.

Therapeutic conjugates of the invention are obtained by coupling a vascular smooth muscle binding protein to a therapeutic agent. In the therapeutic conjugate the vascular - smooth muscle binding protein performs the function of targeting the therapeutic conjugate to vascular smooth muscle cells or pericytes, and the therapeutic agent performs the function of inhibiting the cellular activity of the smooth muscle cell or pericyte.

Therapeutic dosage forms of the present invention exhibit the capability to deliver therapeutic agent to target cells over a sustained period of time. Therapeutic dosage forms of this aspect of the present invention may be of any configuration suitable for this purpose. Preferred therapeutic dosage forms exhibit one or more of the following characteristics:
- microparticulate (e.g., from about 0.5 micrometers to about 100 micrometers in diameter, with from about 0.5 to about 2 micrometers more preferred) or nanoparticulate (e.g., from about 1.0 nanometer to about 1000 nanometers in diameter, with from about 50 to about 250 nanometers more preferred), free flowing powder structure;
- biodegradable structure designed to biodegrade over a period of time between from about 3 to about 180 days, with from about 10 to about 21 days more preferred, or non-biodegradable structure to allow therapeutic agent diffusion to occur over a time period of between from about 3 to about 180 days, with from about 10 to about 21 days preferred;
- biocompatible with target tissue and the local physiological environment into which the dosage form is being administered, including biocompatible biodegradation products ;
- facilitate a stable and reproducible dispersion of therapeutic agent therein, preferably to form a therapeutic agent-polymer matrix, with active therapeutic agent release occurring through one or both of the following routes: (1) diffusion of the therapeutic agent through the dosage form (when the therapeutic agent is soluble in the polymer or polymer mixture forming the dosage form); or (2) release of the therapeutic agent as the dosage form biodegrades; and
- capability to bind with one or more cellular and/or interstitial matrix epitopes, with from about 1 to about 10,000 binding protein/peptide-dosage form bonds preferred and with a maximum of about 1 binding peptide-dosage form per 150 square angstroms of particle surface area more preferred. The total number bound depends upon the particle size used. The binding proteins or peptides are capable of coupling to the particulate therapeutic dosage form through covalent ligand sandwich or non-covalent modalities as set forth herein.

Nanoparticulate therapeutic dosage forms of preferred embodiments of the present invention are biodegradable and bind to the vascular smooth muscle cells and enter such cells primarily by endocytosis. The biodegradation of such nanoparticulates occurs over time (e.g., 10 to 21 days) in prelysosomic vesicles and lysosomes. The preferred larger microparticulate therapeutic dosage forms of the present invention bind to the target cell surface or interstitial matrix, depending on the binding protein or peptide selected, and release the therapeutic agents for subsequent target cell uptake with only a few of the smaller microparticles entering the cell by phagocytosis. A practitioner in the art will appreciate that the precise mechanism by which a target cell assimilates and metabolizes a dosage form of the present invention depends on the morphology, physiology and metabolic processes of those cells.

The size of the targeted therapeutic particulate dosage forms is also important with respect to the mode of cellular assimilation. For example, the smaller nanoparticles can flow with the interstitial fluid between cells and penetrate the infused tissue until it binds to the normal or neoplastic tissue that the binding protein/peptide is selected to target. This feature is important, for example, because the nanoparticles follow lymphatic drainage channels from infused primary neoplastic foci, targeting metastatic foci along the lymphatic tract. The larger microparticles tend to be more easily trapped interstitially in the infused primary tissue.

Preferable dosage forms of the present invention are biodegradable microparticulates or nanoparticulates. More preferably, biodegradable microparticles or nanoparticles are formed of a polymer containing matrix that biodegrades by random, nonenzymatic, hydrolytic scissioning to release therapeutic agent, thereby forming pores within the particulate structure.

Polymers derived from the condensation of alpha hydroxycarboxylic acids and related lactones are preferred for use in the present invention. Such a preferred moiety is formed of a mixture of thermoplastic polyesters (e.g., polylactide or polyglycolide) or a copolymer of lactide and glycolide components, such as poly(lactide-co-glycolide). An exemplary structure, a random poly(DL-lactide-co-glycolide), is shown below, with the values of x and y being manipulable by a practitioner in the art to achieve desirable microparticulate or nanoparticulate properties.

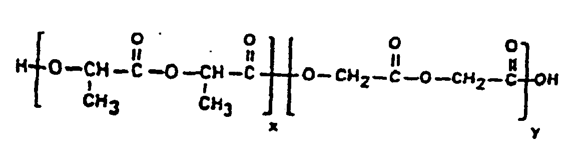

Other agents suitable for forming particulate dosage forms of the present invention include polyorthoesters and polyacetals (Polymer Letters, 18:293, 1980) and polyorthocarbonates (U.S. Patent No. 4,093,709) and the like.

Preferred lactic acid/glycolic acid polymer containing matrix particulates of the present invention are prepared by emulsion-based processes that constitute modified solvent extraction processes such as those described by Cowsar et al., "'Poly(Lactide-Co-Glycolide) Microcapsules for Controlled Release of Steroids, "Methods Enzymology, 112:101-116, 1985 (steroid entrapment in microparticulates): Eldridge et al., "Biodegradable and Biocompatible Poly(DL-Lactide-Co-Glycolide) Microspheres as an Adjuvant for Staphylococcal Enterotoxin B Toxoid Which Enhances the Level of Toxin-Neutralizing Antibodies," Infection and Immunity, 59:2978-2986, 1991 (toxoid entrapment); Cohen et al., "controlled Delivery Systems for Proteins Based on Poly(Lactic/Glycolic Acid) Microspheres," Pharmaceutical Research, 8(6):713-720, 1991 (enzyme entrapment); and Sanders et al., "Controlled Release of a Luteinizing Hormone-Releasing Hormone Analogue from Poly (D,L-Lactide-Co-Glycolide) Microspheres, " J. Pharmaceutical Science, 73(9):1294-1297, 1984 (peptide entrapment).

In general, the procedure for forming particulate dosage forms of the present invention involves dissolving the polymer in a halogenated hydrocarbon solvent, dispersing a therapeutic agent solution (preferably aqueous) therein, and adding an additional agent that acts as a solvent for the halogenated hydrocarbon solvent but not for the polymer. The polymer precipitates out from the polymer-halogenated hydrocarbon solution onto droplets of the therapeutic agent containing solution and entraps the therapeutic agent. Preferably the therapeutic agent is substantially uniformly dispersed within the dosage form of the present invention. Following particulate formation, they are washed and hardened with an organic solvent. Water washing and aqueous non-ionic surfactant washing steps follow, prior to drying at room temperature under vacuum.

For biocompatibility purposes, particulate dosage forms, characterized by a therapeutic agent dispersed therein in matrix form, are sterilized prior to packaging, storage or administration. Sterilization may be conducted in any convenient manner therefor. For example, the particulates can be irradiated with gamma radiation, provided that exposure to such radiation does not adversely impact the structure or function of the therapeutic agent dispersed in the therapeutic agent-polymer matrix or the binding protein/peptide attached thereto. If the therapeutic agent or binding protein/peptide is so adversely impacted, the particulate dosage forms can be produced under sterile conditions.

Release of the therapeutic agent from the particulate dosage forms of the present invention can occur as a result of both diffusion and particulate matrix erosion. Biodegradation rate directly impacts therapeutic agent release kinetics. The biodegradation rate is regulable by alteration of the composition or structure of the dosage form. For example, alteration of the lactide/glycolide ratio in preferred dosage forms of the present invention can be conducted as described by Tice et al., "Biodegradable Controlled-Release Parenteral Systems," Pharmaceutical Technology, pp. 26-35, 1984; by inclusion of polymer hydrolysis modifying agents such as citric acid and sodium carbonate as described by Kent et al., "Microencapsulation of Water Soluble Active Polypeptides," U.S. Patent No. 4,675,189; and by variation of particulate size as described by Beck et al., "Poly(DL-Lactide-Co-Glycolide)/Norethisterone Microcapsules: An Injectable Biodegradable Contraceptive," Biol. Reprod., 28:186-195, 1983 or the like. All of the aforementioned methods of regulating biodegradation rate influence the intrinsic viscosity of the polymer containing matrix, thereby altering the hydration rate thereof.

The preferred lactide/glycolide structure is biocompatible with the mammalian physiological environment. Also, these preferred dosage forms have the advantage that biodegradation thereof forms lactic acid and glycolic acid, both normal metabolic products of mammals.

Functional groups required for binding protein/peptide-dosage form bonding to the particles, are optionally included in the particulate structure along with the non-degradable or biodegradable polymeric units. Functional groups that are exploitable for this purpose include those that are reactive with peptides, such as carboxyl groups, amine groups, sulfhydryl groups and the like. Preferred binding enhancement moieties include the terminal carboxyl groups of the preferred (lactide-glycolide) polymer containing matrix or the like.

Useful vascular smooth muscle binding protein is a polypeptide, peptidic, or mimetic compound (as described below) that are capable of binding to a target or marker on the surface of a vascular smooth muscle cell in such a manner that when internalized by the cell, the binding protein distributes into an intracellular compartment permitting delivery of the therapeutic agent. Representative examples of useful vascular smooth muscle binding proteins include antibodies (e.g., monoclonal and polyclonal affinity-purified antibodies, F(ab')₂, Fab', Fab, and Fv fragments and/or complementary determining regions (CDR) of antibodies or functional equivalents thereof, (e.g., binding peptides and the like); growth factors, cytokines, and polypeptide hormones and the like; and, macromolecules recognizing extracellular matrix receptors (e.g., integrin and fibronectin receptors and the like).

Other preferred binding peptides useful in targeting the dosage form embodiment of the present invention include those that localize to intercellular stroma and matrix located between and among vascular smooth muscle cells. Such binding peptides deliver the therapeutic agent to the interstitial space between the target cells. The therapeutic agent is released into such interstitial spaces for subsequent uptake by the vascular smooth muscle cells. Preferred binding peptides of this type are associated with epitopes on collagen, extracellular glycoproteins such as tenascin, reticulum and elastic fibers and other intercellular matrix material.

Preferred tumor cell binding peptides are associated with epitopes of myc, ras, bcr/Ab1, erbB and like gene products as well as mucins, cytokine receptors such as IL-6, EGF, TGF and the like, which localize to certain lymphomas (myc), carcinomas such as colon cancer (ras), carcinoma (erbB), adenocarcinomas (mucins), breast cancer and hepatoma (IL-6 receptor), breast cancer (EGF and TGF), respectively. Preferred immune system effector cells binding peptides are anti-TAC, IL-2 and the like, which localize to activated T cells and macrophages, respectively. Other preferred binding proteins/peptides useful in the practice of the present invention include moieties capable of localizing to pathologically proliferating normal tissues, such as pericytes of the intraocular vasculature implicated in degenerative eye disease.

Therapeutic agents of the invention are selected to inhibit a cellular activity of a vascular smooth muscle cell, e.g., proliferation, migration, increase in cell volume, increase in extracellular matrix synthesis (e.g., collagens, proteoglycans, and the like) or secretion of extracellular matrix materials by the cell. Preferably, the therapeutic agent acts either: a) as a "cytostatic agent" to prevent or delay cell division in proliferating cells by inhibiting replication of DNA (e.g., a drug such as adriamycin), or by inhibiting spindle fiber formation (e.g., a drug such as colchicine) and the like; or b) as an inhibitor of migration of vascular smooth muscle cells from the medial wall into the intima, e.g., an "anti-migratory agent"; or c) as an inhibitor of the intracellular increase in cell volume (i.e., the tissue volume occupied by a cell, "cytoskeletal inhibitor" or "metabolic inhibitor"); or d) as an inhibitor that blocks cellular protein synthesis and/or secretion or organization of extracellular matrix (i.e., an "anti-matrix agent").

Representative examples of "cytostatic agents" include, e.g., modified toxins, methotrexate, adriamycin, radionuclides (e.g., such as disclosed in Fritzberg et al., U.S. Patent No. 4,897,255), protein kinase inhibitors, inhibitors of specific enzymes such as the nuclear enzyme DNA topoisomerase II and DNA polymerase, RNA polymerase, adenyl guanyl cyclase, superoxide dismutase inhibitors, terminal deoxynucleotidyl-transferase, reverse transcriptase, antisenseoligonucleotides that suppress smooth muscle cell proliferation and the like, which when delivered into a cellular compartment at an appropriate dosage will act to impair proliferation of a smooth muscle cell or pericyte without killing the cell. Other examples of "cytostatic agents" include peptidic or mimetic inhibitors (i.e., antagonists, agonists, or competitive or non-competitive inhibitors) of cellular factors that may (e.g., in the presence of extracellular matrix) trigger proliferation of smooth muscle cells or pericytes: e.g., cytokines (e.g., interleukins such as IL-1), growth factors, (e.g., PDGF, TGFalpha or beta, tumor necrosis factor, smooth muscle- and endothelial-derived growth factors, i.e., endothelin, FGF), homing receptors (e.g., for platelets or leukocytes), and extracellular matrix receptors (e.g., integrins). Representative examples of useful therapeutic agents in this category of cytostatic agents for smooth muscle proliferation include: subfragments of heparin, Triazolopryimidine (Trapidil; a PDGF antagonist), Lovastatin, and Prostaglandins E1 or I2.

Representative examples of "anti-migratory agents" include inhibitors (i.e., agonists and antagonists, and competitive or non-competitive inhibitors) of chemotactic factors and their receptors (e.g., complement chemotaxins such as C5a, C5a desarg or C4a; extracellular matrix factors, e.g., collagen degradation fragments), or intracellular cytoskeletal proteins involved in locomotion (e.g., actin, cytoskeletal elements, and phosphatases and kinases involved in locomotion). Representative examples of useful therapeutic agents in this category of anti-migratory agents include: caffeic acid derivatives and Nilvadipine (a calcium antagonist), and steroid hormones.

Representative examples of "cytoskeletal inhibitors" include colchicine, vinblastin, and the like that act on microtubule and microfilament networks within a cell.

Representative examples of "metabolic inhibitors" include trichothecenes, and modified diphtheria and ricin toxins, *Pseudomonas exotoxin* and the like. In a preferred embodiment the therapeutic conjugate is constructed with a therapeutic agent that is a simple trichothecene or a macrocyclic trichothecene, e.g., a verrucarin or roridin. Trichothecenes are drugs produced by soil fungi of the class *Fungi imperfecti* or isolated from *Baccharus megapotamica* (Bamburg, J.R. Proc. Molec. Subcell. Biol. 8:41-110, 1983; Jarvis & Mazzola, Acc. Chem. Res. 15:338-395, 1982). They appear to be the most toxic molecules that contain only carbon, hydrogen and oxygen (Tamm, C. Fortschr. Chem. Org. Naturst. 31:61-117, 1974). They are all reported to act at the level of the ribosome as inhibitors of protein synthesis at the initiation, elongation, or termination phases.

There are two broad classes of trichothecenes: those that have only a central sesquiterpenoid structure and those that have an additional macrocyclic ring (simple and macrocyclic trichothecenes, respectively). The simple trichothecenes may be subdivided into three groups (i.e., Group A, B, and C) as described in U.S. Patent Nos. 4,744,981 and 4,906,452 Representative examples of Group A simple trichothecenes include: Scirpene, Roridin C, dihydrotrichothecene, Scirpen-4, 8-diol, Verrucarol, scirpentriol, T-2 tetraol, pentahydroxyscirpene, 4-deacetylneosolaniol, trichodermin, deacetylcalonectrin, calonectrin, diacetylverrucarol, 4-monoacetoxyscirpenol, 4,15-diacetoxyscirpenol, 7-hydroxydiacetoxyscirpenol, 8-hydroxydiacetoxy-scirpenol (Neosolaniol), 7, 8-dihydroxydiacetoxyscirpenol 7-hydroxy-8-acetyldiacetoxyscirpenol, 8-acetylneosolaniol, NT-1, NT-2, HT-2, T-2, and acetyl T-2 toxin.

Representative examples of Group B simple trichothecenes include: Trichothecolone, Trichothecin, deoxynivalenol, 3-acetyldeoxynivalenol, 5-acetyldeoxynivalenol, 3,15-diacetyldeoxynivalenol, Nivalenol, 4-acetylnivalenol (Pusarenon-X), 4,15-idacetylnivalenol, 4,7, 15-triacetylnivalenol, and tetra-acetylnivalenol. Representative examples of Group C simple trichothecenes include: Crotocol and Crotocin. Representative macrocyclic trichothecenes include verrucarin A, verrucarin B, Verrucarin J (Satratoxin C), Roridin A, Roridin D, Roridin E (Satratoxin D), Roridin H, Satratoxin F, Satratoxin G, Satratoxin H, Vertisporin, Mytoxin A, Mytoxin C, Mytoxin B, Myrotoxin A, Myrotoxin B, Myrotoxin C, Myrotoxin D, Roritoxin A, Roritoxin B, and Roritoxin D. In addition, the general "trichothecene" sesquiterpenoid ring structure is also present in compounds termed "baccharins" isolated from the higher plant *Baccharis megapotamica,* and these are described in the literature, for instance as disclosed by Jarvis et al. (Chemistry of Alleopathy, ACS Symposium Series No. 268: ed. A.C. Thompson, 1984, pp. 149-159).

Representative examples of "anti-matrix agents" include inhibitors (i.e., agonists and antagonists and competitive and non-competitive inhibitors) of matrix synthesis, secretion and assembly, organizational cross-linking (e.g., transglutaminases cross-linking collagen), matrix remodeling (e.g., following wound healing). A representative example of a useful therapeutic agent in this category of anti-matrix agents is colchicine, an inhibitor of secretion of extracellular matrix.

For the sustained release dosage form embodiments of the present invention, therapeutic agents preferably are those that inhibit vascular smooth muscle cell activity without killing the cells (i.e., cytostatic therapeutic agents). Preferred therapeutic agents for this purpose exhibit one or more of the following capabilities: to inhibit DNA synthesis prior to protein synthesis inhibition or to inhibit migration of vascular smooth muscle cells into the intima. These therapeutic agents do not significantly inhibit protein synthesis (i.e., do not kill the target cells) and, therefore, facilitate cellular repair and matrix production to stabilize the vascular wall lesion caused by angioplasty, by reducing smooth muscle cell proliferation.

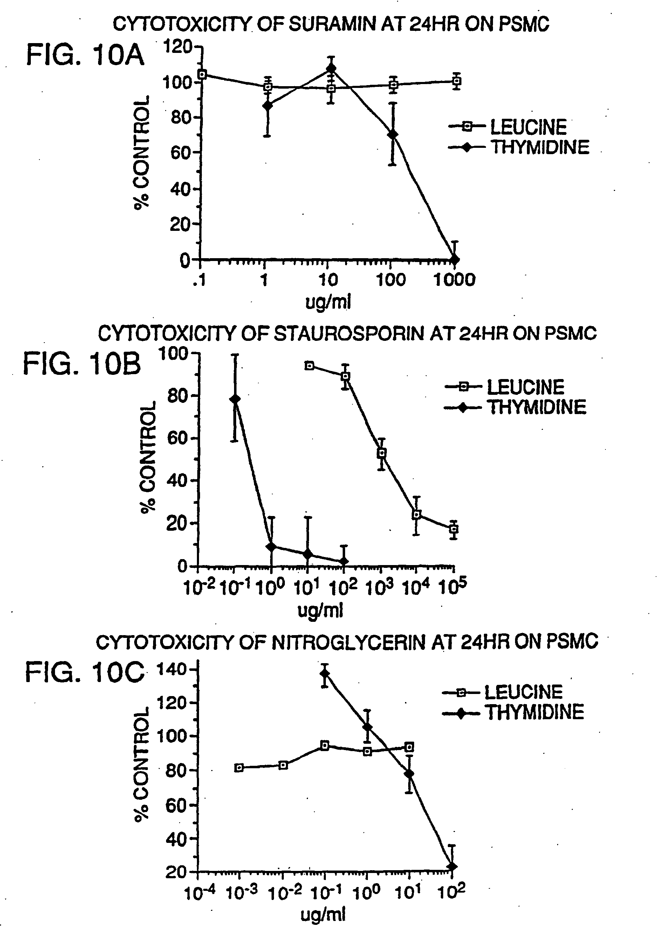

Exemplary of such preferred therapeutic agents are protein kinase inhibitors, such as staurosporin (Sigma Chemical, St. Louis, Missouri), and suramin (FBA Pharmaceuticals, West Haven, Connecticut) as well as nitroglycerin (DuPont Pharmaceuticals, Inc., Manuti, Puerto Rico) or analogs or functional equivalents thereof. These compounds are cytostatic and have been shown to exert minimal protein synthesis inhibition and cytotoxicity at concentrations where significant DNA synthesis inhibition occurs (see Example 8 and Figs. 10A-10C). A useful protocol for identifying therapeutic agents useful in sustained release dosage form embodiments of the present invention is set forth in Example 8. A practitioner in the art is capable of designing substantially equivalent experimental protocols for making such an identification for different target cell populations such as adherent monolayer target cell types.

Other embodiments of the present invention involve agents that are cytotoxic to cancer cells. Preferred agents for these embodiments are Roridin A, *Pseudomonas exotoxin* and the like or analogs or functional equivalents thereof. A plethora of such therapeutic agents, including radioisotopes and the like, have been identified and are known in the art. In addition, protocols for the identification of cytotoxic moieties are known and employed routinely in the art.

Modulation of immune system-mediated disease effector cells can also be accomplished using the sustained release dosage forms of the present invention. Such modulation is preferably conducted with respect to diseases having an effector cell population that is accessible through local dosage form administration. Therapeutic moieties having the requisite modulating activity, e.g., cytocidal, cytostatic, metabolism modulation or like activity upon lymphorecticular cells in the treatment of arthritis (intra-articular administration), sprue (oral administration), uveitis and endophthalmitis (intra-ocular administration and keratitis (sub-conjunctival administration), are identifiable using techniques that are known in the art. Preferred agents for these embodiments include Roridin A, *Pseudomonas exotoxin,* suramin, protein kinase inhibitors (e.g., staurosporin) and the like or analogs or functional equivalents thereof.

Other preferred therapeutic agents useful in the practice of the present invention include moieties capable of reducing or eliminating pathological proliferation of normal tissues. Exemplary of such therapeutic agents are those capable of reducing or eliminating pathological proliferation of pericytes of the intraocular vasculature implicated in degenerative eye disease.

Vascular smooth muscle binding proteins of the invention bind to targets on the surface of vascular smooth muscle cells. It will be recognized that specific targets, e.g., polypeptides or carbohydrates, proteoglycans and the like that are associated with the cell membranes of vascular smooth muscle cells are useful for selecting (e.g., by cloning) or constructing (e.g., by genetic engineering or chemical synthesis) appropriately specific vascular smooth muscle binding proteins. Particularly useful "targets" are internalized by smooth muscle cells, e.g., as membrane constituent antigen turnover occurs in renewal. Internalization by cells may also be by mechanisms involving phagolysosomes, clathrin-coated pits, receptor-mediated redistribution or endocytosis and the like. In a preferred embodiment, such a "target" is exemplified by chondroitin sulfate proteoglycans (CSPGs) synthesized by vascular smooth muscle cells and pericytes, and a discrete portion (termed an epitope herein) CSPG molecule having an apparent molecular weight of about 250kD is especially preferred. The 250kD target is an N-linked glycoprotein that is a component of a larger 400kD proteoglycan complex (14). In one presently preferred embodiment of the invention, a vascular smooth muscle binding protein is provided by NR-AN-01 monoclonal antibody (a subculture of NR-ML-05) that binds to an epitopes in a vascular smooth muscle CSPG target molecule. The monoclonal antibody designated NR-ML-05 reportedly binds a 250kD CSPG synthesized by melanoma cells (Morgan et al., U.S. Pat. No. 4,897,255). Smooth muscle cells and pericytes also reportedly synthesize a 250kD CSPG as well as other CSPGs (11). NR-ML-05 binding to smooth muscle cells has been disclosed (Fritzberg et al., U.S. Pat. No. 4, 879, 225). Monoclonal antibody NR-ML-05 and subculture NR-ML-05 No. 85-41-4I-A2, freeze A2106, have both been deposited with the American Type Culture Collection, Rockville, MD and granted Accession Nos. HB-5350 and HB-9350, respectively. NR-ML-05 is the parent of, and structurally and functionally equivalent to, subclone NR-AN-01, disclosed herein. It will be recognized that NR-AN-01 is just one example of a vascular smooth muscle binding protein that specifically associates with the 400kD CSPG target, and that other binding proteins associating with this target and other epitopes in this target (14) are also useful in the therapeutic conjugates and methods of the invention. In the present case, six other murine monoclonal antibodies and two human chimeric monoclonal antibodies have also been selected as described herein that specifically target to the 250kD CSPG of vascular smooth muscle cells. The antibodies also appear to be internalized by the smooth muscle cells following binding to the cell membrane. Immunoreactivity studies have also shown the binding of the murine MAbs to the 250kD antigen in 45 human normal tissues and 30 different neoplasms and some of these results have been disclosed previously (U.S. Patent No. 4,879,225). In this disclosure and other human clinical studies, MAbs directed to the CSPG 250kD antigen localized to vascular smooth muscle cells *in vivo.* Further, it will be recognized that the amino acid residues involved in the multi-point kinetic association of the NR-AN-01 monoclonal antibody with a CSPG marker antigenic epitope, (i.e., the amino acids constituting the complementary determining regions), are determined by computer-assisted molecular modeling and by the use of mutants having altered antibody binding affinity. The binding-site amino acids and three dimensional model of the NR-AN-01 antigen binding site serve as a molecular model for constructing functional equivalents, e.g., short polypeptides ("minimal polypeptides") that have binding affinity for a CSPG synthesized by vascular smooth muscle cells and pericytes.

In a presently preferred embodiment for treating stenosis following vascular surgical procedures, e.g., PTCA, selected binding proteins, e.g. antibodies or fragments, for use in the practice of the invention having a binding affinity of >10⁴ liter/mole for the vascular smooth muscle 250kD CSPG, and also the ability to be bound to and internalized by smooth muscle cells or pericytes.

Three-dimensional modeling is also useful to construct other functional equivalents that mimic the binding of NR-AN-01 to its antigenic epitope, e.g., "mimetic" chemical compounds that mimic the three-dimensional aspects of NR-AN-01 binding to its epitope in a CSPG target antigen. As used herein "minimal polypeptide" refers to an amino acid sequence of at least six amino acids in length. As used herein, the term "mimetic" refers to an organic chemical polymer constructed to achieve the proper spacing for binding to the amino acids of an NR-AN-01 CSPG target synthesized by vascular smooth muscle cells or pericytes.

It will be recognized that the inventors also contemplate the utility of human monoclonal antibodies or "humanized" murine antibody as a vascular smooth muscle protein in the therapeutic conjugates of their invention. For example, murine monoclonal antibody may be "humanized" by genetically recombining nucleotide sequence encoding the murine Fv region (i.e., containing the antigen binding sites) with the nucleotide sequence encoding a human constant domain region and an Fc region, e.g., in a manner similar to that disclosed in European Patent Application No. 0,411,893 A2. Humanized vascular smooth muscle binding partners will be recognized to have the advantage of decreasing the immunoreactivity of the antibody or polypeptide in the host recipient, which may thereby be useful for increasing the half-life and reducing the possibility of adverse immune reactions.

Also contemplated as useful binding peptides for restenosis treatment dosage forms of the present invention are those that localize to intercellular stroma and matrix located between and among vascular smooth muscle cells. Such binding peptides deliver the therapeutic agent to the interstitial space between the target cells. The therapeutic agent is released into such interstitial spaces for subsequent uptake by the vascular smooth muscle cells. Preferred binding peptides of this type are associated with epitopes on collagen, extracellular glycoproteins such as tenascin, reticulum and elastic fibers, cytokeratin and other intercellular matrix components. Minimal peptides, mimetic organic chemical compounds, human or humanized monoclonal antibodies and the like that localize to intracellular stroma and matrix are also useful as binding peptides in this embodiment of the present invention. Such binding peptides may be identified and constructed or isolated in accordance with known techniques. In preferred embodiments of the present invention, the interstitial matrix binding protein binds to a target epitope with an association constant of at least about 10⁻⁴M. Useful binding peptides for cancer treatment embodiments of the present invention include those associated with cell membrane and cytoplasmic epitopes of cancer cells and the like. These binding peptides localize to the surface membrane of intact cells and internal epitopes of disrupted cells, respectively, and deliver the therapeutic agent for assimilation into the target cells. Minimal peptides, mimetic organic compounds and human or humanized antibodies that localize to the requisite tumor cell types are also useful as binding peptides of the present invention. Such binding peptides may be identified and constructed or isolated in accordance with known techniques. Preferred binding peptides of these embodiments of the present invention bind to a target epitope with an association constant of at least about 10⁻⁶M.

Binding peptides to membrane and cytoplasmic epitopes and the like that localize to immune system-mediated disease effector cells, e.g., cells of the lymphoreticular system, are also useful to deliver sustained release dosage forms of the present invention. The therapeutic agent is delivered to target cells for internalization therein by such sustained release dosage forms. Minimal peptides, mimetic organic compounds and human or humanized antibodies that localize to the requisite effector cell types are also useful as binding peptides of the present invention. Such binding peptides may be identified and constructed or isolated in accordance with known techniques. Preferred binding peptides of these embodiments of the present invention bind to a target epitope with an association constant of at least about 10⁻⁶M.

Other preferred binding proteins or peptides useful in the practice of the present invention include moieties capable of localizing to pathologically proliferating normal tissues, such as pericytes of the intraocular vasculature implicated in degenerative eye disease. The therapeutic agent is delivered to target cells for internalization therein by such sustained release dosage forms. Minimal peptides, mimetic organic compounds and human or humanized antibodies that localize to the requisite effector cell types are also useful as binding peptides of the present invention. Such binding peptides may be identified and constructed or isolated in accordance with known techniques. Preferred binding peptides of these embodiments of the present invention bind to a target epitope with an association constant of at least about 10⁻⁶M.

Representative "coupling" methods for linking the therapeutic agent through covalent or non-covalent bonds to the vascular smooth muscle binding protein include chemical cross-linkers and heterobifunctional cross-linking compounds (i.e., "linkers") that react to form a bond between reactive groups such as hydroxyl, amino, amido, or sulfhydryl groups in a therapeutic agent and other reactive groups (of a similar nature) in the vascular smooth muscle binding protein. This bond may be for example a peptide bond, disulfide bond, thioester bond, amide bond, thioether bond, and the like. In one illustrative example, conjugates of monoclonal antibodies with drugs have been summarized by Morgan and Foon (Monoclonal Antibody Therapy to cancer: Preclinical Models and Investigations, Basic and Clinical *Tumor Immunology, Vol. 2*, Kluwer Academic Publishers, Hingham, MA) and by Uhr *J. of Immunol*. 133:i-vii, 1948). In another illustrative example where the conjugate contains a radionuclide cytostatic agent, U.S. Patent No. 4,897,255, Fritzberg et al., is instructive of coupling methods that may be useful. In one presently preferred embodiment, the therapeutic conjugate contains a vascular smooth muscle binding protein coupled covalently to a trichothecene drug. In this case, the covalent bond of the linkage may be formed between one or more amino, sulfhydryl, or carboxyl groups of the vascular smooth muscle binding protein and a) the trichothecene itself; b) a trichothecene hemisuccinate carboxylic acid; c) a trichothecene hemisuccinate (HS) N-hydroxy succinimidate ester; or d) trichothecene complexes with poly-L-lysine or any polymeric carrier. Representative examples of coupling methods for preparing therapeutic conjugates containing a trichothecene therapeutic agent are described in U.S. Patents No. 4,906,452 and 4,744,981. Other examples using a hydrazide for forming a Schiff base linkage between binding proteins and tricothecenes are disclosed in pending U.S. patent application No. 07/415, 154, incorporated herein by reference.

The choice of coupling method will be influenced by the choice of vascular smooth muscle binding protein or peptide, interstitial matrix binding protein or peptide and therapeutic agent and also by such physical properties as, e.g., shelf life stability and/or such biological properties as, e.g., half-life in cells and blood, intracellular compartmentalization route, and the like. For example, in one presently preferred therapeutic conjugate, hemisuccinate conjugates of the Roridin A therapeutic agent have a longer serum half-life than those of Verrucarin A, and this increased stability results in a significantly increased biological activity.

The sustained release embodiment of the present invention includes a therapeutic agent dispersed within a non-biodegradable or biodegradable polymeric structure. Such dispersion is conducted in accordance with the procedure described by Cowsar et al., "Poly(Lactide-Co-Glycolide) Microcapsules for Controlled Release of Steroids," Methods Enzymology, 112:101-116, 1985; Eldridge et al., "Biodegradable and Biocompatible Poly(DL-Lactide-Co-Glycolide) Microspheres as an Adjuvant for Staphylococcal Enterotoxin B Toxoid Which Enhances the Level of Toxin-Neutralizing Antibodies," Infection and Immunity, 59 : 2978-2986, 1991 ; Cohen et al., "Controlled Delivery Systems for Proteins Based on Poly(Lactic/Glycolic Acid) Microspheres," Pharmaceutical Research, 8(6):713-720, 1991; and Sanders et al., "Controlled Release of a Luteinizing Hormone-Releasing Hormone Analogue from Poly(D,L-Lactide-Co-Glycolide) Microspheres," J. Pharmaceutical Science, 73(9):1294-1297, 1984.

The physical and chemical character of the sustained release dosage form of the present invention are amenable to several alternative modes of attachment to binding proteins or peptides. Dosage forms of the present invention are capable of binding to binding proteins/peptides through, for example, covalent linkages, intermediate ligand sandwich attachment, non-covalent adsorption or partial entrapment. When the preferred poly-lactic/glycolic acid particulates are formed with the therapeutic agent dispersed therein, the uncharged polymer backbone is oriented both inward (with the quasi lipophilic therapeutic agent contained therein) and outward along with a majority of the terminal carboxy groups. These surface carboxy groups may serve as covalent attachment sites when activated by, for example, a carbodiimide, for nucleophilic groups of the binding protein/peptide. Such nucleophilic groups include lysine epsilon amino groups (amide linkage), serine hydroxyl groups (ester linkage) or cysteine mercaptan groups (thioester linkage). Reactions with particular groups depend upon pH and the reduction state of the reaction conditions.

For example, poly-lactic/glycolic acid particulates having terminal carboxylic acid groups are reacted with N-hydroxybenztriazole in the presence of a water soluble carbodiimide of the formula R-N=C=N-R' (wherein R is a 3-dimethyaminopropyl group or the like and R' is an ethyl group or the like). The benztriazole-derivatized particulates (i.e., activated imidate-bearing moieties) are then reacted with a protein/peptide nucleophilic moiety such as an available epsilon amino moiety. Alternatively, p-nitrophenol, tetrafluorophenol, N-hydroxysuccinimide or like molecules are useful to form an active ester with the terminal carboxy groups of poly-lactic/glycolic acid particulates in the presence of carbodiimide. Other binding protein/peptide nucleophilic moieties include hydroxyl groups of serine, endogenous free thiols of cysteine, thiol groups resulting from reduction of binding protein/peptide disulfide bridges using reducing agents commonly employed for that purpose (e.g., cysteine, dithiothreitol, mercaptoethanol and the like) and the like. Additionally, the terminal carboxy groups of the poly lactic/glycolic acid particulates are activatable by reaction with thionyl chloride to form an acyl chloride derivatized moiety. The derivatized particulates are then reacted with binding peptide/protein nucleophilic groups to form targeted dosage forms of the present invention.

Direct dosage form-binding protein or peptide conjugation may disrupt binding protein/peptide target cell recognition. Ligand sandwich attachment techniques are useful alternatives to achieve dosage form-binding protein/peptide attachment. Such techniques involve the formation of a primary peptide shell using a protein that does not bind to the target cell population. Binding protein/peptide is then bound to the primary peptide shell to provide the resultant particulate with functional binding protein/peptide. An exemplary ligand sandwich approach involves covalent attachment of avidin or streptavidin to the particulates through functional groups as described above with respect to the "direct" binding approach. The binding protein or peptide is derivatized, preferably minimally, with functionalized biotin (e.g., through active ester, hydrazide, iodoacetal, maleimidyl or like functional groups). Ligand (i.e., binding peptide/functionalized biotin) attachment to the available biotin binding sites of the avidin/streptavidin primary protein shell occurs through the use of a saturating amount of biotinylated protein/peptide.

For example, poly-lactic/glycolic acid particulates having terminal carboxylic acid groups are activated with carbodiimide and subsequently reacted with avidin or streptavidin. The binding protein or peptide is reacted with biotinamidocaproate N-hydroxysuccinimide ester at a 1-3 molar offering of biotin-containing compound to the binding protein/peptide to form a biotinylated binding protein/peptide. A molar excess of the biotinlyated binding protein/peptide is incubated with the avidin-derivatized particulates to form a targeted dosage form of the present invention.

Alternatively, the particulate carboxy groups are biotinylated (e.g., through carbodiimide activation of the carboxy group and subsequent reaction with amino alkyl biotinamide). The biotinylated particulates are then incubated with a saturating concentration (i.e., a molar excess) of avidin or streptavidin to form protein coated particulates having free biotin binding sites. Such coated particulates are then capable of reaction with a molar excess of biotinylated binding protein formed as described above, the prepared as described above. Another option involves avidin or streptavidin bound binding peptide attachment to biotinylated particulates.

In addition, binding protein/peptide-particulate attachment can be achieved by adsorption of the binding peptide to the particulate, resulting from the nonionic character of the partially exposed polymer backbone of the particulate. Under high ionic strength conditions (e.g., 1.0 molar NaCl), hydrogen and hydrophobic particulate-binding protein/peptide binding are favored.

Moreover, binding protein/peptide may be partially entrapped in the particulate polymeric matrix upon formation thereof. Under these circumstances, such entrapped binding protein/peptide provides residual selective binding character to the particulate. Mild particulate formation conditions, such as those employed by Cohen et al., Pharmaceutical Research, 8: 713-720 (1991), are preferred for this embodiment of the present invention. Such entrapped binding protein is also useful in target cell reattachment of a partially degraded particulate that has undergone exocytosis. Other polymeric particulate dosage forms (e.g., non-biodegradable dosage forms) having different exposed functional groups can be bound to binding proteins or peptides in accordance with the principles discussed above.

Exemplary non-biodegradable polymers useful in the practice of the present invention are polystyrenes, polypropylenes, styrene acrylic copolymers and the like. Such non-biodegradable polymers incorporate or can be derivatized to incorporate functional groups for attachment of binding protein/peptide, including carboxylic acid groups, aliphatic primary amino groups, aromatic amino groups and hydroxyl groups.

Carboxylic acid fictional groups are coupled to binding protein or peptide using, for example, the reaction mechanisms set forth above for poly-lactic/glycolic acid biodegradable polymeric particulate dosage forms. Primary amino functional groups are coupled by, for example, reaction thereof with succinic anhydride to form a terminal carboxy moiety that can be bound to binding peptide/protein as described above. Additionally, primary amino groups can be activated with cyanogen bromide and form guanidine linkages with binding protein/peptide primary amino groups. Aromatic amino functional groups are, for example, diazotized with nitrous acid to form diazonium moieties which react with binding protein/peptide tyrosines, thereby producing a diazo bond between the non-biodegradable particulate and the binding protein/peptide. Hydroxyl functional groups are coupled to binding protein/peptide primary amino groups by, for example, converting the hydroxyl moiety to a terminal carboxylic acid functional group. Such a conversion can be accomplished through reaction with chloroacetic acid followed by reaction with carbodiimide. Sandwich, adsorption and entrapment techniques discussed above with respect to biodegradable particulates are analogously applicable to non-biodegradable particulate-binding protein/peptide affixation.

In a preferred embodiment, targeting is specific for potentially proliferating cells that result in increased smooth muscle in the intimal region of traumatized vascular site, e.g., following angioplasty, e.g., pericytes and vascular smooth muscle cells. Aspects of the invention relate to therapeutic modalities in which the therapeutic conjugate of the invention is used to delay, reduce, or eliminate smooth muscle proliferation after angioplasty, e.g., PTCA, atherectomy and percutaneous transluminal coronary rotational atheroblation.

Targeting can also be specific for primary or metastatic tumor foci accessible to local administration, e.g., tumors exposed for infiltration by laparotomy or visible for fluoroscopic or computerized tomography guiding and infusion needle administration to internal tumor foci or tumors confined to a small area or cavity within the mammal, e.g., ovarian cancer located in the abdomen. Aspects of this approach involve therapeutical modalities wherein the therapeutic agent is cytotoxic to the target cells or metabolically modulates the cells, increasing their sensitivity to chemotherapy and/or radiation therapy.

Targeting can also be specific for a local administration accessible effector cell population implicated in immune system-mediated diseases, e.g., arthritis, intra-ocular immune system-mediated disease or sprue. Aspects of this approach involve therapeutic modalities wherein the therapeutic agent is cytotoxic or modifies the biological response of the target cells to affect a therapeutic objective.

Targeting can also be specific for a local administration accessible pathologically proliferating normal cell population implicated in, e.g., degenerative eye disease. Aspects of this approach involve therapeutic modalities wherein the therapeutic agent reduces or eliminates proliferation of the target cell population.

For treatment of a traumatized or diseased vascular site, the therapeutic conjugates or dosage forms of the invention may be administered to the host using an infusion catheter, such as produced by C.R. Bard Inc., Billenca, MA., or that disclosed by Wolinsky (7; U.S. Patent No. 4,824,436) or Spears (U.S. Patent No. 4,512,762). In this case, a therapeutically effective dosage of the therapeutic conjugate will be typically reached when the concentration of conjugate in the fluid space between the balloons of the catheter is in the range of about 10⁻³ to 10⁻¹²M. It will be recognized from the Examples provided herewith that therapeutic conjugates of the invention may only need to be delivered in a therapeutic dosage sufficient to expose the proximal (6 to 9) cell layers of the intimal cells lining the lumen to the therapeutic conjugate, and further that this dosage can be determined empirically, e.g., by a) infusing vessels from suitable animal model systems and using immunohistochemical methods to detect the conjugate and its effects (e.g., such as exemplified in the EXAMPLES below); and b) conducting suitable *in vitro* studies such as exemplified in EXAMPLES 3, 4, and 5, below). In a representative example, this therapeutically effective dosage is achieved by preparing 10 ml of a 200 µg/ml therapeutic conjugate solution wherein the vascular smooth muscle protein binding protein is NR-AN-01 and the therapeutic agent is Roridin A, a trichothecene drug. For treating vascular trauma, e.g., resulting from surgery or disease (e.g., see below), when the therapeutic conjugate is administered with an infusion catheter, 10 ml will commonly be sufficient volume to fill the catheter and infuse 1 to 1.5 ml into one to three traumatic lesion sites in the vessel wall. It will be recognized by those skilled in the art that desired therapeutically effective dosages of a therapeutic conjugate according to the invention will be dependent on several factors, including, e.g.: a) the binding affinity of the vascular smooth muscle binding protein in the therapeutic conjugate; b) the atmospheric pressure applied during infusion; c) the time over which the therapeutic conjugate administered resides at the vascular site; d) the nature of the therapeutic agent employed; and/or e) the nature of the vascular trauma and therapy desired. Those skilled practitioners trained to deliver drugs at therapeutically effective dosages, (e.g., by monitoring drug levels and observing clinical effects in patients) will determine the optimal dosage for an individual patient based on experience and professional judgement. In a preferred embodiment, about 0.3 atm (i.e., 300 mm of Hg) to about 3 atm of pressure applied for 15 seconds to 3 minutes directly to the vascular wall is adequate to achieve infiltration of a therapeutic conjugate containing the NR-AN-01 binding protein into the smooth muscle layers of a mammalian artery wall. Those skilled in the art will recognize that infiltration of the therapeutic conjugate into intimal layers of a diseased human vessel wall will probably be variable and will need to be determined on an individual basis.

Sustained release dosage forms of an embodiment of the invention may only need to be delivered in a therapeutic dosage sufficient to expose the proximal (6 to 9) cell layers of the tunica media smooth muscle cells lining the lumen to the dosage form. This dosage is determinable empirically, e.g., by a) infusing vessels from suitable animal model systems and using immunohistochemical, fluorescent or electron microscopy methods to detect the dosage form and its effects; and b) conducting suitable *in vitro* studies. In a representative example, this therapeutically effective dosage is achieved by determining in smooth muscle cell tissue culture the pericellular agent dosage, which at a continuous exposure results in a therapeutic effect between the toxic and minimal effective doses. This therapeutic level is obtained *in vivo* by determining the size, number and therapeutic agent concentration and release rate required for particulates infused between the smooth muscle cells of the artery wall to maintain this pericellular therapeutic dosage. The dosage form should release the therapeutic agent at a rate that approximates the pericellular dose of the following exemplary therapeutic agents: from about 0.1 to about 0.01 micrograms/ml nitroglycerin, from about 1.0 to about 1000 micrograms/ml of suramin and from about 0.1 to about 10⁵ nanograms/ml of staurosporin.

It will be recognized by those skilled in the art that desired therapeutically effective dosages of the dosage form of the invention will be dependent on several factors, including, e.g.: a) the binding affinity of the binding protein associated with the dosage form; b) the atmospheric pressure and duration of the infusion; c) the time over which the dosage form administered resides at the target site; d) the rate of therapeutic agent release from the particulate dosage form; e) the nature of the therapeutic agent employed; f) the nature of the trauma and/or therapy desired; and/or g) the intercellular and/or intracellular localization of the particulate dosage form. Those skilled practitioners trained to deliver drugs at therapeutically effective dosages, (e.g., by monitoring therapeutic agent levels and observing clinical effects in patients) are capable of determining the optimal dosage for an individual patient based on experience and professional judgement. In a preferred embodiment, about 0.3 atm (i.e., 300 mm of Hg) to about 3 atm of pressure applied for 15 seconds to 3 minutes to the arterial wall is adequate to achieve infiltration of a dosage form bound to the NR-AN-01 binding protein into the smooth muscle layers of a mammalian artery wall. Wilensky et al., "Direct Intraarterial Wall Injection of Microparticles Via a Catheter: A Potential Drug Delivery Strategy Following Angioplasty," Am. Heart Jour., 122 (4): 1136-1140, 1991. Those skilled in the art will recognize that infiltration of a sustained release dosage form into a target cell population will probably be variable and will need to be determined on an individual basis.

It will also be recognized that the selection of a therapeutic agent that exerts its effects intracellularly, e.g., on ribosomes or DNA metabolism, will influence the dosage and time required to achieve a therapeutically effective dosage and that this process can be modeled *in vitro* and in animal studies, such as those described in the Examples provided below, to find the range of concentrations over which the therapeutic conjugate or dosage form should be administered to achieve its effects of delaying, reducing or preventing restenosis following angioplasty. For example, therapeutic conjugates radiolabeled with alpha-, beta- or gamma-emitters of known specific activities (e.g., millicuries per millimole or milligram of protein) are useful for determining the therapeutically effective dosage by using them in animal studies and human trials with quantitative imaging, or autoradiography of histological tissue sections to determine the concentration of therapeutic conjugate that is required by the therapeutic protocol. A therapeutically effective dosage of the therapeutic conjugate or dosage form will be reached when at least three conditions are met: namely, (1) the therapeutic conjugate or dosage form is distributed in the intimal layers of the traumatically injured vessel; (2) the therapeutic conjugate or dosage form is distributed within the desired intracellular compartment of the smooth muscle cells, i.e., that compartment necessary for the action of the therapeutic agent, or the therapeutic agent released from the dosage form extracellularly is distributed within the relevant intracellular compartment; and (3) the therapeutic agent inhibits the desired cellular activity of the vascular smooth muscle cell, e.g., proliferation, migration, increased cellular volume, matrix synthesis, and the like described above.

It will be recognized that where the therapeutic conjugate or dosage form is to be delivered with an infusion catheter, the therapeutic dosage required to achieve the desired inhibitory activity for a therapeutic conjugate or dosage form can also be anticipated through the use of *in vitro* studies. In a preferred aspect, the infusion catheter may be conveniently a double balloon or quadruple balloon catheter with a permeable membrane. In one representative embodiment, a therapeutically effective dosage of a therapeutic conjugate or dosage form is useful in treating vascular trauma resulting from disease (e.g., atherosclerosis, aneurysm, or the like) or vascular surgical procedures such as angioplasty, arthrectomy, placement of a stent (e.g., in a vessel), thrombectomy, and grafting. Arthrectomy may be performed, for example, by surgical excision, ultrasound or laser treatment, or by high pressure fluid flow. Grafting may be, for example, vascular grafting using natural or synthetic materials or surgical anastomosis of vessels such as, e.g., during organ grafting. Those skilled in the art will recognize that the appropriate therapeutic dosage for a given vascular surgical procedure (above) is determined in *in vitro* and *in vivo* animal model studies, and in human preclinical trials. In the EXAMPLES provided below a therapeutic conjugate containing Roridin A and NR-AN-01 achieved a therapeutically effective dosage *in vivo* at a concentration which inhibited cellular protein synthesis in test cells *in vitro* by at least 5 to 50% as judged by incorporation of radiolabeled amino acids.

In the case of therapeutic agents of conjugates or dosage forms containing anti-migratory or anti-matrix therapeutic agents, cell migration and cell adherence in *in vitro* assays, respectively, may be used for determining the concentration at which a therapeutically effective dosage will be reached in the fluid space created by the infusion catheter in the vessel wall.

While one representative embodiment of the invention relates to therapeutic methods employing an infusion catheter, it will be recognized that other methods for drug delivery or routes of administration may also be useful, e.g., injection by the intravenous, intralymphatic, intrathecal, or other intracavity routes. For intravenous administration, nanoparticulate dosage forms of the present invention are preferred. Intravenous administration of nanoparticulates is useful, for example, where vascular permeability is increased in tumors for leakage, especially in necrotic areas of tumors having damaged vessels which allow the leakage of particles into the interstitial fluid, and where artery walls have been denuded and traumatized allowing the particles to enter the interstitial area of the tunica media.

Advantageously, non-coupled vascular smooth muscle cell binding protein (e.g., free NR-AN-01 antibody) is administered prior to administration of the therapeutic agent conjugate or dosage form to provide a blocker of non-specific binding to cross-reactive sites. Blocking of such sites is important because vascular smooth muscle cell binding proteins will generally have some low level of cross-reactivity with cells in tissues other than the desired smooth muscle cells. Such blocking can improve localization of the therapeutic conjugate or dosage form at the specific vascular site, e.g., by making more of the therapeutic conjugate available to the cells. As an example, non-coupled vascular smooth muscle binding protein is administered from about 5 minutes to about 48 hours, most preferably from about 5 minutes to about 30 minutes, prior to administration of the therapeutic conjugate or dosage form. In one preferred embodiment of the invention, the unlabeled specific "blocker" is a monovalent or bivalent form of an antibody (e.g., a whole antibody or an F(ab)'_{2'} Fab, Fab', or Fv fragment of an antibody). The monovalent form of the antibody has the advantage of minimizing displacement of the therapeutic conjugate or dosage form while maximizing blocking of the non-specific cross-reactive sites. The non-coupled vascular smooth muscle cell binding protein is administered in an amount effective to blocking binding of a least a portion of the non-specific cross-reactive sites in a patient. The amount may vary according to such factors as the weight of the patient and the nature of the binding protein. In general, about 0.06 mg to 0.20 mg per kg body weight or more of the unlabeled specific blocker is administered to a human.

In addition, a second irrelevant vascular smooth muscle cell binding protein may optionally be administered to a patient prior to administration of the therapeutic conjugate or dosage form to reduce non-specific binding of the therapeutic conjugate or dosage form to tissues. In a preferred embodiment, the irrelevant binding protein may be an antibody which does not bind to sites in the patient through antigen-specific binding, but instead binds in a non-specific manner, e.g., through Fc receptor binding reticuloendothelial cells, asialo-receptor binding, and by binding to ubiquitin-expressing cells. The irrelevant "blocker" decreases non-specific binding of the therapeutic conjugate or dosage form and thus reduces side-effects, e.g., tissue toxicity, associated with the use of the therapeutic conjugate or dosage form. The irrelevant "blocker" is advantageously administered from 5 minutes to 48 hours, most preferably from 15 minutes to one hour, prior to administration of the therapeutic conjugate or dosage form, although the length of time may vary depending upon the therapeutic conjugate and route or method of injection. Representative examples of irrelevant "blockers" include antibodies that are nonreactive with human tissues and receptors or cellular and serum proteins prepared from animal sources that when tested are found not to bind in a specific manner (e.g., with a Ka<10³M⁻¹) to human cell membrane targets.

It will be recognized that the conjugates and dosage forms of the invention are not restricted in use for therapy following angioplasty; rather, the usefulness of the therapeutic conjugates and dosage forms will be proscribed by their ability to inhibit cellular activities of smooth muscle cells and pericytes in the vascular wall. Thus, other aspects of the invention include therapeutic conjugates and dosage forms and protocols useful in early therapeutic intervention for reducing, delaying, or eliminating (and even reversing) atherosclerotic plaques and areas of vascular wall hypertrophy and/or hyperplasia. Therapeutic conjugates and dosage forms of the invention also find utility for early intervention in pre-atherosclerotic conditions, e.g., they are useful in patients at a high risk of developing atherosclerosis or with signs of hypertension resulting from atherosclerotic changes in vessels or vessel stenosis due to hypertrophy of the vessel wall.

The therapeutic conjugates and dosage forms of the invention may also be used in therapeutic modalities for enhancing the regrowth of endothelial cells in injured vascular tissues and in many kinds of wound sites including epithelial wounds. In these therapeutic modalities, the therapeutic conjugates and dosage forms of the invention find utility in inhibiting the migration and/or proliferation of smooth muscle cells or pericytes. Smooth muscle cells and pericytes have been implicated in the production of-factors *in vitro* that inhibit endothelial cell proliferation and their proliferation can also result in a physical barrier to establishing a continuous endothelium. Thus, the therapeutic conjugates and dosage forms of the invention find utility in promoting neo-angiogenesis, e.g., during wound healing, vessel grafts and following vascular surgery.

Still other aspects of the invention relate to therapeutic modalities for enhancing wound healing in a vascular site and improving the structural and elastic properties of healed vascular tissues. In these therapeutic modalities using the therapeutic conjugate or dosage form of the invention, i.e., to inhibit the migration and proliferation of smooth muscle cells or pericytes in a vessel wall, improves the strength and quality of healing of the vessel wall. Smooth muscle cells in the vascular wound site contribute to the normal process of contraction of the wound site which promotes wound healing. It is presently believed that migration and proliferation of smooth muscle cells may detract from this normal process and impair the long-term structural and elastic qualities of the healed vessel. Thus, other aspects of the invention provide for therapeutic conjugates and dosage forms that inhibit smooth muscle and pericyte proliferation and migration and improve the quality of the healed vasculature.

Methods for the treatment of cancer and immune system-mediated diseases through local administration of a targeted particulate dosage form can also benefit from the concepts described herein. The particulate dosage form is, for example, administered locally into primary and/or metastatic foci of cancerous target cells. Local administration is preferably conducted using a infusion needle or intraluminal administration route, infusing the particulate dosage form in the intercellular region of the tumor tissue or in luminal fluid surrounding the tumor cells.

Primary foci introduction is preferably conducted with respect to target cells that are generally situated in confined areas within a mammal, e.g., ovarian carcinomas located in the abdominal cavity. The dosage form of the present invention binds to the target cell population and, optionally, is internalized therein for release of the therapeutic agent over time. Local administration of dosage forms of the present invention to such primary foci results in a localized effect on such target cells with limited exposure of other sensitive organs, e.g., the bone marrow and kidneys, to the therapeutic agent.

When metastatic foci constitute the target cell population, the administered microparticles and larger nanoparticles are primarily bound to the target cells situated near the infusion site and are, optionally, internalized for release of the therapeutic agent, thereby generating a marked and localized effect on the target cells immediately surrounding the infusion site. In addition, smaller nanoparticles follow interstitial fluid flow or lymphatic drainage channels and bind to target cells that are distal to the infusion site and undergoing lymphatic metastasis.

The targeted dosage forms can be used in combination with more commonly employed immunoconjugate therapy. In this manner, the immunoconjugate achieves a systemic effect within the limits of systemic toxicity, while the dosage form of the present invention delivers a concentrated and sustained dose of therapeutic agent to the primary and metastatic foci which often receive an inadequate therapeutic dose from such "systemic" immunoconjugate administration alone and avoids or minimizes systemic toxic effects.

Where the target cell population can be accessed by local administration, the dosage forms of the present invention are utilized to control immune system-mediated diseases. Exemplary of such diseases are arthritis, sprue, uveitis, endophthalmitis, keratitis and the like. The target cell populations implicated in these embodiments of the present invention are confined to a body cavity or space such as joint capsules, pleural and abdominal cavity, eye and sub-conjunctual space, respectively. Local administration is preferably conducted using the infusion needle for a intrapleural, intraperitoneal, intraocular or sub-conjunctual administration route.

This embodiment of the present invention provides a more intense, localized modulation of immune system cells with minimal effect on the systemic immune system cells. Optionally, the systemic cells of the immune system are simultaneously treatable with a chemotherapeutic agent conjugated to a binding protein or peptide. Such a conjugate preferably penetrates from the vascular lumen into target immune system cells.

The local particulate dosage form administration may also localize to normal tissues that have been stimulated to proliferate, thereby reducing or eliminating such pathological proliferation. An example of this embodiment of the present invention involves intraocular administration of a particulate dosage form coated with a binding protein or peptide-that localizes to pericytes of neovascularizing tissue. Proliferation of these pericytes causes degenerative eye disease. Preferred dosage forms of the present invention release compounds capable of suppressing the pathological proliferation of the target cell population.

The invention will be better understood by making reference to the following specific examples.

### EXAMPLE 1

### Binding to Vascular Smooth Muscle Cells In the Blood Vessel Wall In Vivo.

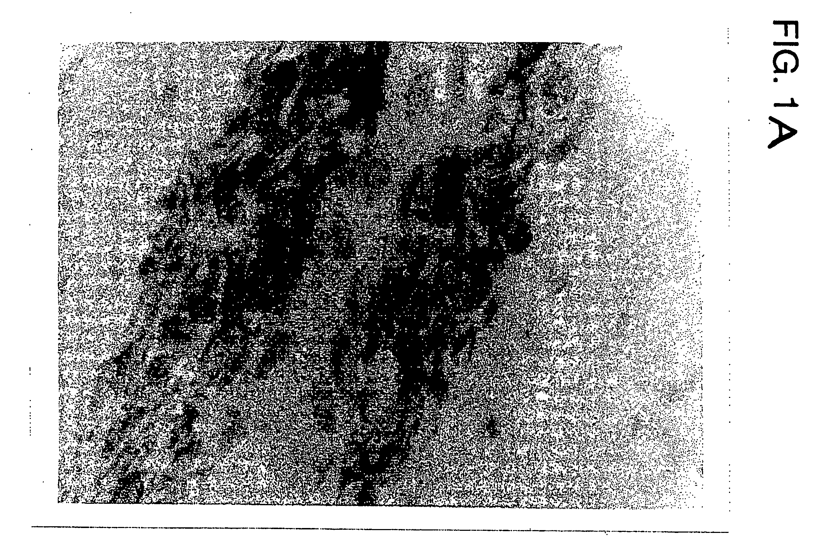

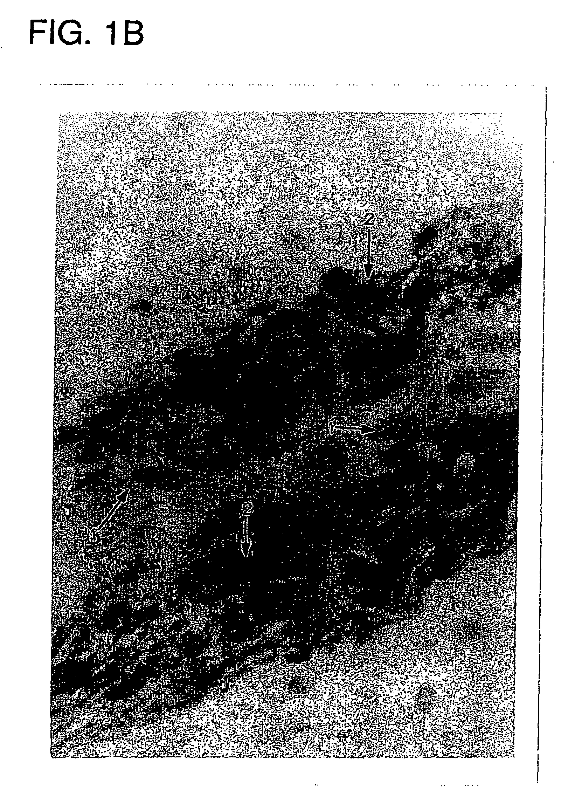

FIGURE 1 illustrate A and B the binding of NR-AN-01 ( a murine IgG2b κ MAb) to the smooth muscle cells in the vascular wall of an artery wall in a normal 24-year old male patient, 4 days after the i.v. administration of NR-AN-01, Fig. 1A is blank, Fig. 1B is marked with identifiers and #2 as described below . FIGURE 1 is a photomicrograph of a histological section taken through the medial region of an arterial wall of the patient after NR-AN-01 administration where the section was reacted *ex vivo* with HRP-conjugated goat anti-mouse IgG. The reaction of the HRP-conjugate with NR-ML-05 MAb was visualized by adding 4-chloro-1-napthol as a peroxidase substrate. The reaction product of the substrate forms an insoluble purple precipitate at the reaction site (shown at #1, FIGURE 1). A counter stain was used to visualize collagenous extracellular matrix material (shown at #2, FIGURE 1). Smooth muscle cells are visualized under microscopic examination as purple stained cells. This photomicrograph demonstrates the ability of the MAb to specifically bind to human vascular smooth muscle *in vivo,* and to be internalized by the cells and remain in the cells for extended periods.

### EXAMPLE 2

### Therapeutic Conjugates containing Trichothecene Therapeutic Agents

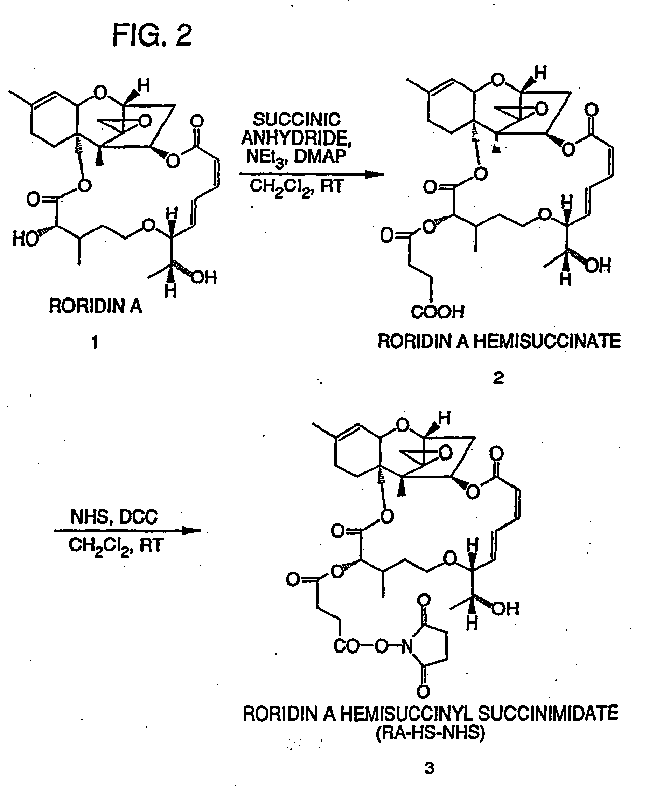

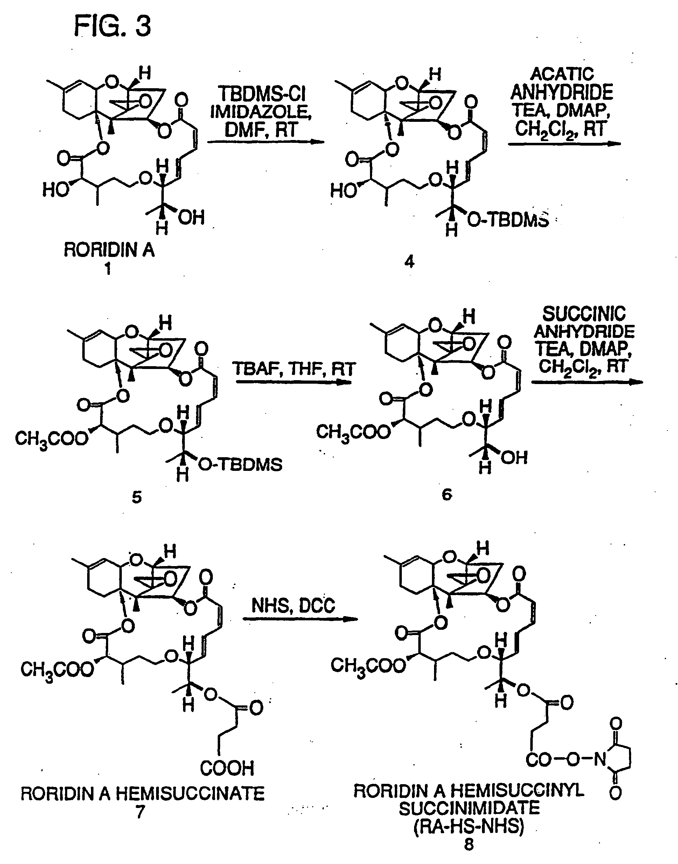

Conjugates of NR-AN-01 and Roridin A were constructed by chemically coupling a hemisuccinate derivative of the trichothecene cytotoxin (as described below) to a monoclonal antibody designated NR-AN-01. Two conjugates were prepared, one coupled at the Roridin A2' position and one at the 13' position. Two schemes were used in this synthesis, as depicted in FIGURE 2 and FIGURE 3. The conjugate was then purified from unreacted Roridin A by PD-10 SEPHAROSE column chromatography (Pharmacia; Piscatawy, N.J.), analyzed by size exclusion high pressure liquid chromatography, and the column fractions were characterized by SDS-PAGE and isoelectric focusing (IEF), as described below.

FIGURE 2 shows diagrammatically the first reaction scheme for synthesis of Roridin A hemisuccinyl succinimidate (RA-HS-NHS) through a two step process with reagents: succinic anhydride, triethylamine (NEt₃) and dimethyl amino pyridine (DMAP) present in dichloromethane (CH₂Cl₂) at room temperature (RT); and, N-hydroxysuccinimide (NHS) and dicyclohexyl carbodiimide (DCC) reagents also in CH₂Cl₂ at RT.

FIGURE 3 shows diagrammatically the second reaction scheme for synthesis of Roridin A hemisuccinyl succinimidate (RA-HS-NHS) through a five step process with reagents: t-butyl dimethyl silyl chloride (TBMS-C1) and imidazole in dimethylformamide (DMF) at room temperature (RT); acetic anhydride, triethylamine (TEA), and diethylaminopyridine in dichloromethane (CH₂Cl₂) at RT; succinic anhydride, triethylamine (TEA) and dimethylaminopyridine (DMAP) in (CH₂Cl₂) at RT; and, N-hydroxysuccinimide (NHS) and dicyclohexyl carbodiimide (DCC) reagents.

### Synthesis of 2' Roridin-A Hemisuccinic Acid (2) :

To 0.5g (0.94 mmol) of Roridin A, 15 ml of dichloromethane was added. To this solution with stirring was added 0.104g (1.04 mmol) of succinic anhydride. To the reaction mixture, 0.2 ml of triethylamine in 5 ml dichloromethane was added. To the homogeneous reaction mixture, a catalytic amount of dimethylaminopyridine was added and stirred at room temperature for 15 hours. Completion of the reaction was followed by thin layer chromatography (CH₂Cl₂ : CH₃OH = 9.7 : 0.3 with few drops of acetic acid). At the end of the reaction, 0.3 ml of glacial acetic acid was added and the solvent removed under reduced pressure. The dried crude residue was partitioned between water and methylene chloride. The combined methylene chloride extracts (3 x 50 ml) were dried over anhydrous sodium sulfate, solvent was removed under vacuum and dried to yield 0.575g (96%) of a crude mixture of three compounds. Preparative C18 HPLC separation of the crude mixture in 50% acetonitride-water with 2% acetic acid yielded 0.36g (60%) of 2 as a white solid.

### Synthesis of Succinimidyl 2' - Roridin A Hemisuccinate (3):

To 0.3g (0.476 mmol) of 2' Roridin A hemisuccinic acid in 30 ml dichloromethane 0.055g (0.478 mmol) N-hydroxysuccinimide was added. To the clear reaction mixture, 0.108 (0.524 mmol) dicyclohexylcarbodiimide was added. The reaction mixture was stirred at room temperature for 6 hours. Completion of the reaction was followed by TLC (CH₂Cl₂: CH₃OH = 9.7 : 0.3 with a few drops of acetic acid) as a developing solvent. A few drops of glacial acetic acid was added to the reaction mixture and the solvent was removed under reduced pressure. To the dried residue dichloromethane was added and the precipitated DCU was filtered. Solvent from the filtrate was removed under reduced pressure to yield a white solid. From the crude product, 0.208g (60%) of 3 was purified by preparative HPLC in 50% acetonitride with 2% acetic acid as a mobile phase.

### Synthesis of 13'-t-Butyldimethylsilyl Roridin A (4):

To 72.3 mg (0.136 mmol) of Roridin A in 0.5 ml dimethylformamide solution, 0.055g (0.367 mmol) t-butyldimethylsilyl chloride and 0.025g (0.368 mmol) of imidazole were added. The reaction mixture was stirred at room temperature for 15 hours. Completion of the reaction was followed by silica gel thin layer chromatography using 1% MeOH-CHCl₃ as a developing solvent. Solvent from the reaction mixture was removed in vacuo and dried. The crude product was partitioned between water and methylene chloride. Solvent from the combined methylene chloride extracts were removed under reduced pressure and dried. The crude product was purified by flash chromatography using EtOAc : Hexane (1:3) as an eluting solvent. Solvent from the eluents were removed under reduced pressure to yield 0.66g (75%) of 4 as a solid.

### Synthesis of 13'-t-Butyldimethylsilyl 2' Acetyl Roridin A (5):

To 0.1g (0.155 mmol) of 13'-t-butyldimethylsilyl Roridin A, in 10 ml dichloromethane, 0.3 ml acetic anhydride, 0.2 ml triethylamine and few crystals of dimethylaminopyridine were added and stored at room temperature for 2 hours. completion of the reaction was followed by TLC in 1% methanol-methylene chloride as a developing solvent. Solvent was removed under reduced pressure and purified by a silica gel column using 1% methanol-chloroform as an elution solvent. Solvent from the eluents was removed under vacuum to yield 0.085g (80%) of 5 as a solid.

### Synthesis of 2' Acetyl Roridin A (6):

To 0.05g (0.073 mmol) of 2' acetyl 13'-t-butyldimethylsilyl Roridin-A in 5 ml tetrahydrofuran, 0.3 ml of 1 M tetrabutyl-ammonium fluoride solution in THF was added. The reaction mixture was stirred at room temperature for 2 hours. Completion of the reaction was followed by silica gel thin layer chromatography using 1% MeOH-CHCl₃ as the developing solvent. Solvent from the reaction mixture was removed under reduced pressure and dried. The crude product was purified on a silica gel column using 1% CH₃OH - CHCl₃ as an eluting solvent. Solvent from the combined eluents were removed under vacuum to yield 0.020g (48%) of 6 as a solid.

### Synthesis of 2'-Acetyl 13'-hemisuccinyl Roridin-A (7):

To 0.05g (0.087 mmol) of 2'-acetyl Roridin-A in 1 ml of dichloromethane, 0.025g (0.25mmol) succinic anhydride and 35ml of triethylamine was added. A few crystals of dimethylaminopyridine was added as a catalyst. The reaction mixture was stirred at room temperature for 24 hours. Completion of the reaction was followed by thin layer chromatography using 5% MeOH-CH₂Cl₂ as developing solvent. At the end of the reaction 30ml of glacial acetic acid was added. solvent from the reaction mixture was removed under reduced pressure and dried. The crude product was partitioned between water and ethyl acetate. Solvent from the combined ethyl acetate fractions were removed under reduced pressure. Crude product was purified by passing through a silica gel column to yield 0.039g (66%) of 7 as a white solid.

### Synthesis of Succinimidyl 2'-Acetyl 13' - Roridin-A Hemisuccinate (1):

To 0.036g (0.0050 mmol) of 2'-acetyl 13'-Roridin A hemisuccinic acid in 2 ml dichloromethane, 0.009g (0.09 mmol) N-hydroxysuccinimide was added. To a stirred solution, 0.012g (0.059 mmol) dicyclohexylcarbodiimide was added. The reaction mixture was stirred at room temperature for 8 hours. Completion of the reaction was followed by silica gel thin layer chromatography using 5% MeOH-CH₂Cl₂ as a developing solvent. A few drops of glacial acetic acid was added to the reaction mixture. Solvent from the reaction mixture was removed under reduced pressure and dried. The crude product was purified on a silica gel column using 5% MeOH-CH₂Cl₂ as an eluting solvent. Solvent from the combined eluents were removed under vacuum to yield 0.025g (61%) of 8 as a white solid.

### Conjugation of Succinimidyl 2'-Roridin-A Hemisuccinate (3) and Succinimidyl 2'-Acetyl 13' -Roridin-A Hemisuccinate (8) to NR-AN-01 Whole Antibody (MAb):

Conjugation reactions were performed at pH 8.0 in borate buffer in the presence of 25% dimethylsulfoxide (DMSO) solvent at room temperature with gentle mixing for 45 minutes prior to purification by gel permeation chromatography. The molar trichothecene drug precursor to antibody offerings were 25:1 and 40:1 for the 2' and 13' Roridin-A analogues (3 and 8), respectively. Antibody concentration was 0.9 to 1.0 mg/ml during the conjugation reaction.

### A Typical 2' Analogue (3) Reaction with 25 mg of Antibody was as follows:

To 4.7 ml of 5.3 mg Ab/ml in phosphate buffered saline, i.e., PBS; 150 mM NaCl, 6.7 mM Phosphate, pH 7.3) was added 10 ml PBS and 5 ml of borate buffer (0.5M, pH 8.0). With stirring gently to the reaction mixture, 6.3 ml of DMSO containing 1.37 mg of succinimidyl 2' Roridin-A hemisuccinate (3) was then added dropwise over a 15 second period.

### Purification:

To purify, one ml reaction aliquots were applied to Pharmacia PD-10 sepharose columns equilibrated in PBS. The eluted conjugate was collected in 2.4 to 4.8 ml fractions. The PD-10 purified conjugate aliquots were then pooled and concentrated on an Amicon PM-10 DiAflo concentrator to 1.5 to 2.0 mg of Ab/ml; sterile filtered through a 0.2µ Gelman Acrodisc^{®} and filled into sterile glass vials in 5 ml volume.

The 2' conjugate was quick frozen in liquid nitrogen and then stored at -70° C until use. The 13' Roridin-A UR-AN-01 conjugate was stored frozen or refrigerated (i.e., 5-10°C).

### Characterization of Conjugates:

Protein concentration was determined by BCA assay using the copper reagent method (Pierce Chemical Corp.).

Assessment of degree of antibody derivatization was performed by first hydrolyzing an aliquot of conjugate in 0.2M carbonate, pH 10.3 for 4 hours (at room temperature for 2' conjugate or at 37°C for the 13' conjugate) followed by filtration through a PM-30 membrane. The filtrate was then assayed for Roridin-A on C-18 reverse Phase HPLC using a mobile phase of 50:48:2 ratio CH₃CN:H₂O:HOAC, respectively. A 1.32 correction factor was used to correct for parallel macrocyclic ring decomposition that gives polar products during the hydrolysis of the 13' conjugate.

Size exclusion chromatography on DuPont Zorbax HPLC and isoelectric focussing using Serva gel plates (pH 3 to 10) were also performed. No indication of aggregation was observed by HPLC.

Immunoassay of the Roridin-A-antibody conjugates were made by either competitive ELISA using biotinylated-Ab with Streptavidin/Peroxidase detection or by a competitive cell binding assay using 125₁₋ labelled antibody. Alternative immunoreactivity was measured under conditions of antigen saturation in a cell binding assay wherein antibody was first trace labeled with 1-125 by the chloramine T method and then subsequently derivatized with 2' and 13' Roridin-A precursors.

### EXAMPLE 3

### Kinetics of Binding to Smooth Muscle Cells

For administration by i.v. catheter, it is desirable that the therapeutic conjugates of the inventiono be administered in less than 3 to 5 minutes so that blood flow can be reestablished in the patient. Therefore, studies were conducted to determine the binding kinetics of a smooth muscle binding protein with a Ka of >10⁹ liter/mole. Because human vascular smooth muscle cells grow slowly in culture, and baboon smooth muscle cells were found to express the human CSPG cell surface marker, BO54 baboon artery smooth muscle cells and human A375 M/M (melanoma; ATCC #CRL1619) cells bearing CSPG surface marker were used in many of the studies described in the Examples, below.

For the kinetic binding studies, A375 M/M and BO54 cells were seeded in sterile 96 well microtiter plates at 2500 cells/well. Plates were wrapped in aluminum foil, and incubated at 37°C overnight in a humidified atmosphere of 5% CO₂/95% air. After approximately 18 hrs. incubation plates were removed, and cells were fixed with 0.05% glutaraldehyde for 5 minutes to prevent membrane turnover. Following fixation, the plates were exhaustively washed with PBS containing 0.5% Tween-20. Serial two-fold dilutions of an NR-AN-01 therapeutic conjugate containing Roridin A were prepared at protein concentrations of 10 mg/ml to 20ng/ml, and each dilution was aliquoted into two wells. The plates were incubated at 4°C with the NR-AN-01 for 5, 15, 30, and 60 minutes after which the unbound protein was removed by aspiration and 100 ml of CS buffer was added (5% chicken serum/ 0.5% Tween-20 in PBS) to each well. CS buffer was removed and the NR-AN-01 therapeutic conjugate bound to the cells was visualized by adding 100 ml of HRP-conjugated goat anti-mouse IgG (Sigma Chemical Co., St. Louis, MO.) to each well; incubating at 4°C for 1 hr.; washing with PBS/0.05% Tween to remove unbound goat IgG; and, adding ABTS chromogenic substrate (i.e., for HRP). After incubating for 30 minutes the amount of NR-AN-01 bound to the cells was quantified by measuring the absorbance at 415nm and 490nm using an ELISA plate reader equipped for data acquisition by a Compaq computer.

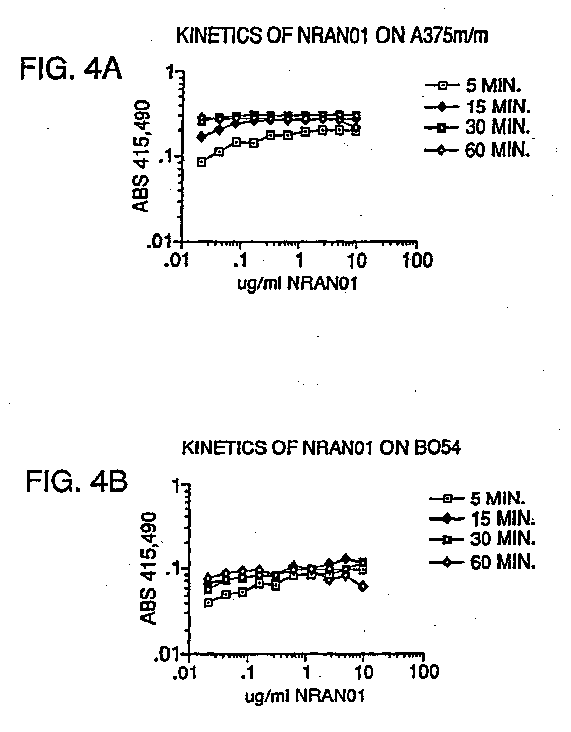

FIGURE 4A graphically depicts the results of *in vitro* studies in which A375m/m marker-positive cells were held at 4°C (i.e., to prevent membrane turnover) for 5 minutes (open squares, FIGURE 4A), 15 minutes (closed diamonds, FIGURE 4A), 30 minutes (closed squares, FIGURE 4A) or 60 minutes (open diamonds, FIGURE 4A) with different concentrations of NR-AN-01 (NRAN01 ug/ml). The binding of the NR-AN-01 MAb to the A375 cells was quantified by washing to remove unbound antibody, adding HRP-conjugated goat anti-mouse IgG to react with the cell-bound MAb, washing to remove unbound goat second antibody, and adding 2,2'-azino-bis(3-ethylbenzthiazoline-6-sulfonic acid) (ABTS) substrate for peroxidase. Color development was monitored after 30 minutes at both 415nm and 490 nm (A415,490).

FIGURE 4B graphically depicts the results of *in vitro* studies conducted in a manner similar to those described above in regard to FIGURE 4A, but using B054 marker-positive smooth muscle cells, i.e., instead of the A375 m/m cells.

The results presented in FIGURE 4A and FIGURE 4B show significant binding of NR-AN-01 to A375 and BO54 cells within 5 minutes at 4°C, even at the lowest dose of 20ng/ml.

### EXAMPLE 4

### Effects of Roridin A and RA-NR-AN-01 Conjugates

The effects of Roridin A (RA) and RA-NR-AN-01 conjugates on cellular protein synthesis, i.e., by ³H-leucine incorporation) and metabolic activity (i.e., by mitochondrial MTT assay) were tested in the experiments detailed in EXAMPLE 5 and EXAMPLE 6, below. The studies in EXAMPLE 4 include experiments to determine the effects of long-term (i.e., 24 hour) treatment with the agents. The studies in EXAMPLE 5 include experiments to determine the effects of "pulse" (i.e., 5 minute) treatment on cells. In both studies the cellular specificity of the effects were evaluated by including "target" cells, (i.e., cells bearing the CSPG "marker"), and non-target cells. For comparative purposes, free-RA (i.e., uncoupled) was also included in the studies. The effects on cellular protein synthesis or metabolic activity were evaluated either immediately following the treatment, or a "recovery period" was allowed, (i.e., involving incubation of the cells overnight at 37°C), to determine the long-term effects of the agents on the cell populations.

### Metabolic Effects After 24 Hours Exposure:

While it is known that monoclonal antibody-drug conjugates may have a degree of specificity for cells bearing marker antigens when employed *in vivo*, it has proven more difficult in many systems to demonstrate *in vitro* specificity of action, especially with-compounds that are Lipophilic. Therefore, the present experiments were conducted in which the inhibitory effects of the NR-AN-01-Roridin A conjugate was tested on target and non-target cells over 24 hours. The results with RN-NR-AN-01-RA were compared to the effect of free Roridin A over the same 24-hour period. A modified methyl-tetrazolium blue (MTT) assay was utilized with [3-(4,5-dimethylthriazol-2-yi)-2,2-diphenyl tetrazolium bromide] to determine cellular metabolic activity. This assay is thought to measure cellular mitochondrial dehydrogenase activity. For some of these studies M14 (melanoma) and BO54 (smooth muscle) cell lines were used as marker-positive target cells and HT29 cells (colon carcinoma; ATCC #HTB38) were used as the non-target specificity control. In other studies A375 was used as marker-positive cells. The HT29 and M14 cells were seeded in 96-well microtiter plates at a concentration of 5.0 x 10³ cells/well, and the B054 cells were seeded at 2.5 x 10³ cells/well. Serial two-fold dilutions of free Roridin A and 2'RA-HS-NR-AN-01 (i.e., Roridin A coupled through a hemisuccinate (HS) coupling agent to the 2' position to NR-AN-01) were prepared in DMEM over a range of protein concentrations from 20 mg/ml to 40pg/ml. Test agents were added (in duplicate) to microtiter wells (100 ml/well), and the plates were wrapped in aluminum foil and incubated at 37°C in a humidified atmosphere consisting of 5% CO₂/95% air for 24 hours. After 24 hours, media was removed (by aspiration), fresh DMEM was added (100 ml/well), and the cells were returned to incubate for an addition overnight (i.e., 16-18 hours) "recovery period". At the end of the "recovery period" cellular metabolic activity was determined by adding 20 ml to each well of a 5mg/ml MTT solution. The plates were covered and incubated at 37°C for 4 hours and then the reaction was developed by adding 100 ml/well of 10% SDS/0.1N HCl. The dark blue solubilized formazan reaction product was developed at room temperature after 16-18 hours and quantified using an ELISA microtiter plate reader at an absorbance of 570nm.

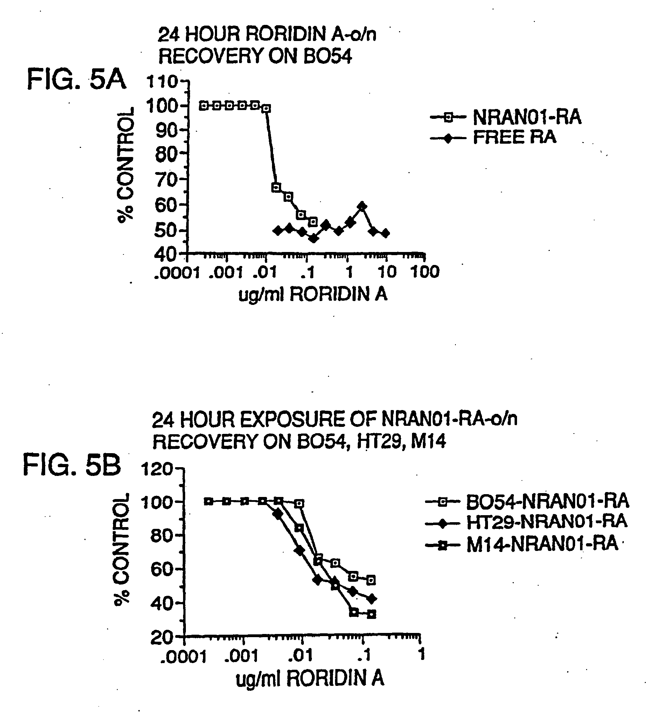

FIGURE 5A graphically depicts the results of *in vitro* studies in which B054 marker-positive smooth muscle cells were incubated with different concentrations of RA-NR-AN-01 (NRAH01-RA; open squares, FIGURE 5A) or free Roridin A (Free RA; closed diamonds, FIGURE 5A) for a period of 24 hours, washed, and then returned to culture for an additional 16-18 hour overnight (o/n) recovery period prior to testing metabolic activity in an MTT assay. The concentrations of Free RA and RA-NR-AN-01 are expressed as the calculated concentration of Roridin A (in mg/ml plotted on a log scale) in the assay (i.e., rather than the total mg/ml of NR-AN-01 protein in the assay) so that direct comparisons could be made. The metabolic activity of the cells in the MTT assay is presented as the percentage of the metabolic activity measured in a control untreated culture of cells (i.e., % control).

FIGURE 5B graphically depicts the results of *in vitro* studies conducted in a manner similar to those described above in regard to FIGURE 5A, but comparing the effects of only RA-NR-AN-01 (NRAN01-RA) on three different cell types: namely, BO54 marker-positive smooth muscle cells (BO54-NRAN01-RA; open squares, FIGURE 5B); HT29 marker-negative control cells (HT29-NRAN01-RA; closed diamonds,FIGURE 5B); and, M14 marker-positive (M14-NRAN01-RA; closed squares, FIGURE 5B). As described above in regard to FIGURE 5A, the concentrations in the present experiment are expressed in terms of ug/ml of Roridin A. Metabolic activity of the cells is expressed in a manner similar to that in FIGURE 5A, i.e., as the percentage of activity measured in an untreated control culture of cells (% control).

The results presented in FIGURE 5A and FIGURE 5B show that metabolic activity measured in the MTT assay was significantly decreased all populations of test cells even 16-18 hours after a 24-hour incubation in either free Roridin A, or the 2' or 13'RA-NR-AN-01 conjugates. The effects of the RA-NR-AN-01-conjugates appeared to be non-specifically inhibitory for both target (BO54 and M14) and non-target (HT29) cells (FIGURES 5A and 5B). The inhibitory effects were observed at a free Roridin A or RA-conjugate concentration of >10ng/ml.

For comparative purposes, a second study was conducted in which the effects of pseudomonas exotoxin-conjugates on cells were evaluated in a similar protocol. For these studies target and non-target cells were treated with PE or PE-NR-AN-01 for 24 hours and then allowed a "recovery period" (as above), before metabolic activity was tested in an MTT assay.

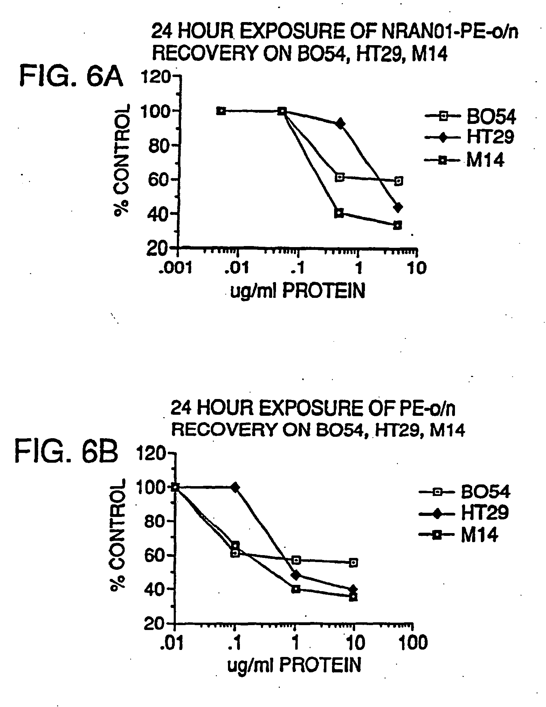

FIGURE 6A graphically depicts the results of *in vitro* studies conducted in a manner similar to those described above in regard to FIGURE 5A, but designed to study the metabolic effects of PE-RA-AN-01 (NRAN01-PE) on cells, i.e., rather than RA-NR-AN-01. Three different cell types were utilized: namely, BO54 marker-positive smooth muscle cells (BO54; open squares, FIGURE 6A); HT29 marker-negative control cells (HT29; closed diamonds, FIGURE 6A); and, M14 maker-positive cells (MT14; closed squares, FIGURE 6A). In this study the concentration of conjugate is expressed in mg/ml NR-AN-01 protein (plotted on a log scale) and the metabolic activity is expressed as the percentage of the MTT activity measured in an untreated control culture (% control).

FIGURE 6 B graphically depicts the results of *in vitro* studies conducted in manner similar to those discussed above in regard to FIGURE 6A, but designed to compare the effects obtained with free PE (PE) to those obtained above, i.e., in FIGURE 6A, with PE-NR-AN-01. The cells, culture conditions, calculations, and presentation of the results is the same as in FIGURE 6A, above.

The results presented in FIGURE 6A and FIGURE 6B show that 24 hours exposure to PE-NR-AN-01 or free PE was non-specifically inhibitory to cells at concentrations of >100ng/ml.

While this type of non-specific inhibition was judged to be of potential value for biological arthrectomy, it was not considered desirable for treatment of restenosis following angioplasty where dead and dying cells may release factors that stimulate smooth muscle proliferation.

### EXAMPLE 5

### Effects of Pulse-Treatment on Cellular Activity

Additional studies were conducted to evaluate the effects of a short-term, i.e. 5 minute, exposure to a Roridin A-containing therapeutic conjugate on cells. In these studies both metabolic activity (measured in MTT assays) and cellular protein synthesis (measured by ³H-leucine incorporation) were evaluated.

### Effects After 5 Minutes of Exposure: Protein Synthesis

The effects of a 5-minute exposure to free Roridin A (RA) or a therapeutic conjugate were evaluated. Roridin A-NR-AN-01 coupled through a hemisuccinyl (HS) at either the 2' position (2'RA-HS-NR-AN-01) or the 13' position (13'RA-HS-NR-AN-01) were employed. (In the case of 13'RA-HS-NR-AN-01 the 2' position of Roridin A was also acetylated.) The RA, 2' or 13'RA-NR-AN-01 conjugates were diluted two fold in sterile DMEM over a range of concentrations from 400ng/ml to 780pg/ml of Roridin A. (The test samples were all normalized to Roridin A so that direct comparisons could be made of the effects at comparable doses.) Samples were aliquoted (in duplicate) into duplicate microtiter plates at 100 ml/well and incubated at room temperature for five minutes.

Both short-term and long-term effects of the test samples on marker-positive A375 and marker-negative HT29 cells was determined. For studying the short-term effects, 100 ml/well of [³H]-leucine (0.5mCi/ml) was immediately added immediately after the 5-minute treatment with conjugate (or RA) and protein synthesis was evaluated over a four-hour period. For determining the long-term effects, the cells were treated for 5 minutes, washed, and then returned to culture for a 24-hour "recovery" period in DMEM medium containing either 5%NBS/5% Serum Plus (i.e., for A375 or HT29 cells) or 10% FBS (i.e., for BO54 cells). At the end of "recovery" period the incubation media was removed (i.e. by aspiration) and ³H-leucine was added (as above). In both cases (i.e., whether short-term or long-term) protein synthesis of the cells was evaluated by incubating the cells with the ³H-leucine for 4 hours at 37°C in a humidified chamber (as above), and all results are calculated by comparison with non-treated cells (i.e., 100% control). After 4 hours the ³H-leucine was removed, the cells were removed from the substrata by trypsin-treatment, aspiration (using a PHD cell harvester) and collected by filtration on glass fiber filters. The glass fiber filters were dried and radioactivity quantified by liquid scintillation spectroscopy in a Beckman Liquid Scintillation Counter.

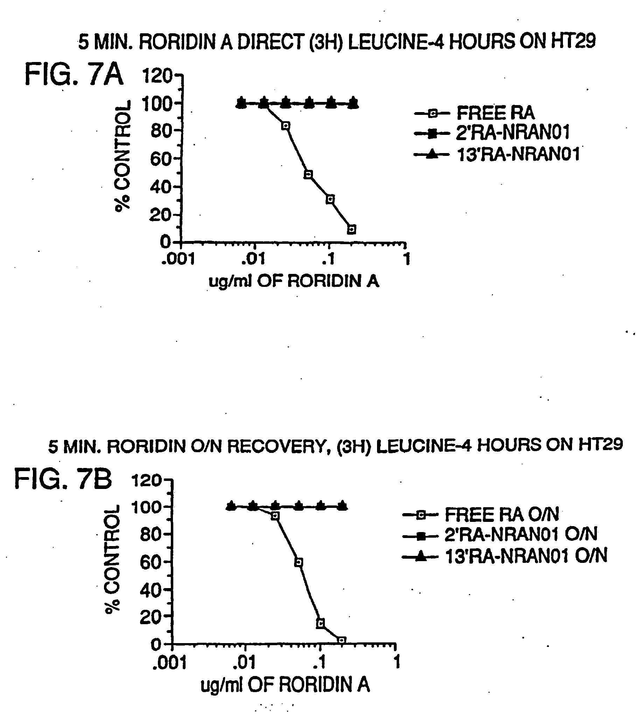

FIGURE 7A graphically depicts the results of *in vitro* studies conducted to investigate the effects on control HT29 marker-negative cells of a 5 minute exposure to different concentrations of Roridin A (Free RA; open squares, FIGURE 7A), or 2'RA-NR-AN-01 (2'RA-NRAN01; closed squares, FIGURE 7A), or 13'RA-NR-AN-01 (13'RA-NRAN01; closed triangles, FIGURE 7A) conjugates. The concentrations of Free RA, 2'RA-NR-AN-01 or 13'NR-AN-01 are expressed as the calculated concentration of Roridin A in the assay (in mg/ml plotted on a log scale), i.e., rather than the total mg/ml of NR-AN-01 protein, so that direct comparisons of the results can be made. For these studies the cells were treated for 5 minutes, washed, and then returned to culture for 4 hours; during which time cellular protein synthesis was evaluated by adding 0.5 mCi/ml of ³H-leucine to the culture medium. At the end of the 4 hour period, cellular proteins were collected and radioactivity was determined. The results are expressed as the percentage of the radioactivity recorded in a control (non-treated) HT29 cell culture (i.e., %control).

FIGURE 7B graphically depicts the results of *in vitro* studies investigating the effects on control HT29 marker-negative cells of a 5 minute expose to different concentrations of Free RA (open squares,FIGURE 7B), 2'RA-NRAN01 ( closed squares, FIGURE 7B), or 13'RA-NRAN01 ( closed triangles, FIGURE 7B), as described above in regard to FIGURE 7A, but in the present experiments the cells were incubated for a 16-18 hour recovery period (i.e., overnight; o/n) prior to testing protein synthesis in a four hour ³H-leucine protein synthesis assay. The results are presented in a manner similar to those above in FIGURE 7A.

The results presented in FIGURE 7A and FIGURE 7B show the short-term and long-term effects, respectively, of RA, 2'RA-HS-NR-AN-01, and 13'RA-HS-NR-AN-01 on protein synthesis by HT29 control cells. The results show a dose-response inhibition of cellular protein synthesis by the free Roridin A, but not by RA-NR-AN-01, in HT29 cells. The inhibition triggered by RA during the 5 minutes of incubation was still manifest after the 16-18 hours recovery period (FIGURE 78). In contrast, treatment of non-target ET29 cells with 2'RA-HS-NR-AN-01 or 13 'RA-HS-NR-AN-01, did not result in detectable inhibition of protein synthesis. Thus, these results (in contrast to those obtained above over 24 hours) seem to suggest a surprising degree of specificity to the *in vitro* action of the NR-AN-01-conjugates when treatment was delivered in a 5-minute "pulse". However, it was also possible that the NR-AN-01-conjugate was inactive, and so additional experiments were conducted to evaluate the effect of the conjugates on target cells.

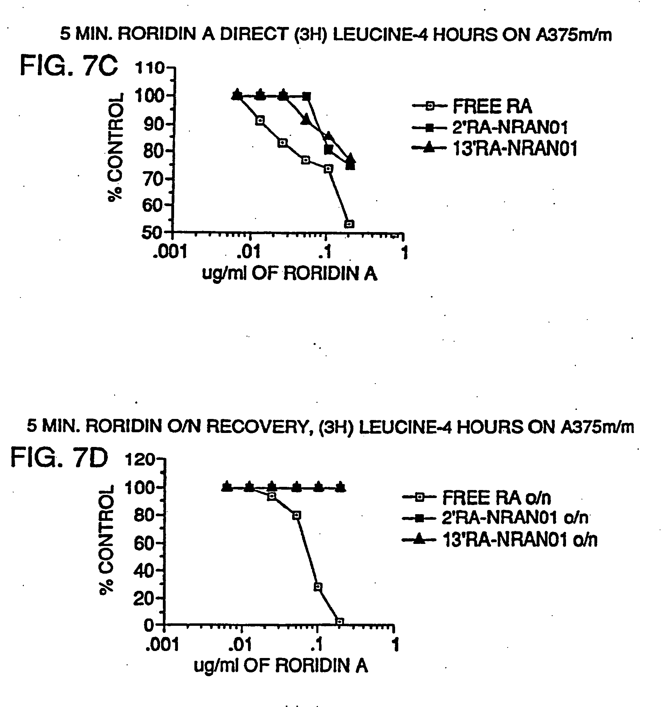

FIGURE 7C graphically depicts the results of *in vitro* studies investigating the effects on A375m/m marker-positive cells of a 5 minute exposure to different concentrations of Free RA ( open squares, FIGURE 7C), 2'RA-NR-AN-01 ( closed squares, FIGURE 7C) or 13' RA-NR-AN-01 ( closed triangles, FIGURE 7C), as described above in regard to FIGURE 7A. In the present studies the A375 cells were incubated for 5 minutes in the test agent, washed, and tested for protein synthesis over the next 4 hours by adding 0.5 mCi/ml ³H-leucine to the culture medium. The results of the experiments are plotted in a manner similar to those described, above, in regard to FIGURE 7A.

FIGURE 7D graphically depicts the results of *in vitro* studies investigating the effects on A375 m/ml marker-positive cells of a 5 minute exposure to different concentrations of Free RA ( open squares ,FIGURE 7D), 2'RA-NRAN01 ( closed squares , FIGURE 7D), 13'RA-NRAN01 (closed triangles, FIGURE 7D), as described above in regard to FIGURE 7B. In the present studies the A375 cells were incubated for 5 minutes in the test agent, washed, and then returned to culture for a 16-18 hour recovery period (i.e., overnight; o/n Recovery) after which time protein synthesis was evaluated during a 4 hour ³H-leucine protein synthesis assay. The results of the experiments are plotted in a manner similar to those described above in regard to FIGURE 7A.

The results presented in FIGURES 7C and FIGURE 7D show the short-term and long-term effects, respectively, of RA, 2'RA-HS-NR-AN-01 and 13'-RA-HS-NR-AN-01 on protein synthesis by A375 target cells. Treatment of target cells with either the 2' or 13' RA-NR-AN-01 therapeutic conjugate resulted in a short-term inhibition of protein synthesis, i.e., observed immediately after the 5-minute pulse treatment (FIGURE 7C). These findings, when combined with the findings in FIGURE 7A and FIGURE 7B, above, suggest that the RA-NR-AN-01 conjugates were active and that they were specifically inhibitory for target cells but not non-target cells. Interestingly, when "pulse" treated target cells were returned to culture no long-term inhibitory effects were observed (FIGURE 7D). The results presented in FIGURES 7C and FIGURE 7D again show that Roridin A is non-specifically inhibitory to test cells (i.e., in a manner similar to FIGURE 7A and FIGURE 7B, above) and that its effects on the cells is manifest even after a 16-18 hour recovery period. Thus, the specific effects of the RA-NR-AN-01 conjugates on target cells during a "pulse" treatment appear to be a property of the NR-AN-01 binding protein.

The results obtained with B054 arterial smooth muscle cells were similar to those obtained with the A375 cells, above, i.e., free Roridin A showed a dose-response inhibition of protein synthesis in the short-term equated to be 60%, 66%, and 90% of control at 200ng/ml, 100ng/ml, and 50ng/ml; and in long-term the effects on protein synthesis were equated to be 27%, 46%, and 98% of control at the same dosages. In contrast, the 2' or 13'RA-NR-AN-01 showed only 10-20% inhibition for short- or long-term effects on protein synthesis (i.e., >80% of control).

Thus, the results show a short-term specific reversible effect of Roridin A-conjugated NR-RA-01 on target cells when delivered as a "pulse" treatment. However, since only protein synthesis was evaluated in these experiments it was possible that cellular metabolic activity might be affected in the cells as a result of the "pulse" treatment. Therefore, additional studies were conducted in which cellular metabolic activity was evaluated following "pulse" treatment.

### Effects After 5 Minutes of Exposure: Metabolic Activity

MTT assays were conducted at 48 hours following a 5-minute exposure of target and non-target cells to RA or RA-NR-AN-01 conjugates. Target cells in these studies included BO54 and A375, and non-target cells included HT29 cells. Sterile 96 well microtiter plates were seeded with 2500 cells/well, wrapped in aluminum foil and incubated in a humidified chamber containing 5% CO₂/95% air for 16-18 hours. Serial two-fold dilutions of Roridin A (RA), 2'RA-HS-NR-AN-01 and 13'RA-HS-NR-AN-01 were prepared from 400 ng/ml to 780pg/ml, and 100 ml aliquots of the dilutions were dispensed into duplicate wells. After 5 minutes exposure to the test samples the cells were washed to remove the test samples, and fresh medium was added. The cells were allowed 48 hours of recovery prior to testing: i.e., plates were incubated for 48 hours, and then cellular metabolic activity was determined by adding 20 ml/well of a 5mg/ml MTT solution. The plates were covered and incubated at 37°C for 4 hours and then the reaction was developed as described above, (see EXAMPLE 4, above). The dark blue solubilized formazan reaction product was developed at room temperature after a 16-18 hour incubation. The samples were quantified using an ELISA microtiter plate reader at an absorbance of 570nm.

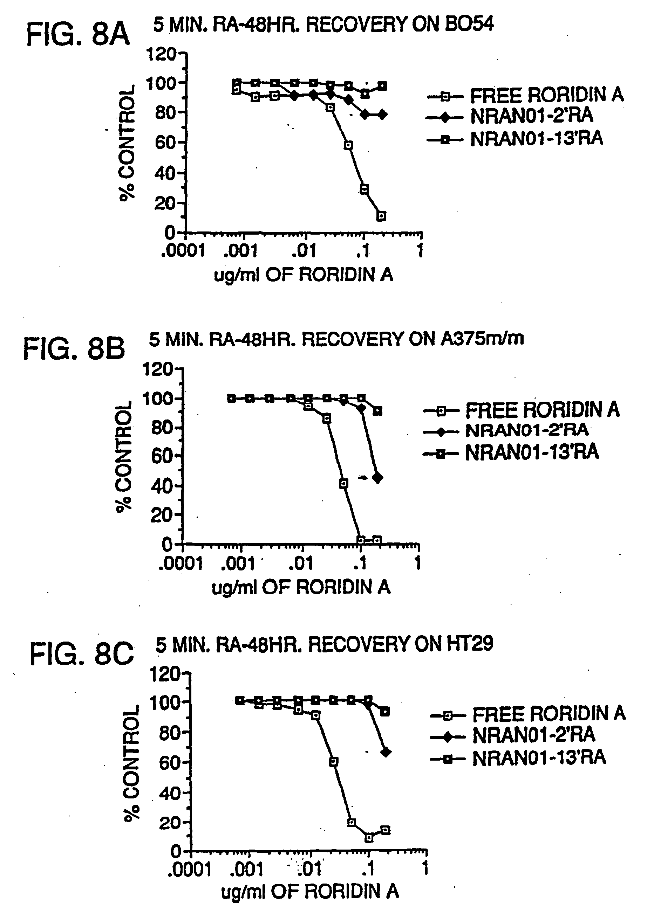

FIGURE 8A graphically depicts the results of *in vitro* studies investigating the effects on BO54 marker-positive smooth muscle cells of a 5 minute exposure to different concentrations of Roridin A ( open squares, FIGURE 8A), 2'RA-NR-AN-01 (NRAN01-2'RA; closed diamonds, FIGURE 8A), or 13' RA-NR-AM-01 (NRAN01-13'RA; closed squares, FIGURE 8A). The experiments were conducted in a manner similar to those described above in regard to FIGURE 7B, but metabolic activity was assayed by MTT assay, i.e. rather than protein synthesis as in FIGURE 7B, and cells were also given 48 hours to recover (rather than 24 hours, as in FIGURE 7B). The results of the experiments are plotted in a manner similar to those described (above) in regard to FIGURE 7A .

FIGURE 8B graphically depicts the results of *in vitro* studies investigating the effects on A375 m/m marker-positive cells of a 5 minute exposure to different concentrations of Roridin A ( open squares, FIGURE 8B), 2'RA-NR-AN-01 (NRAN01-2'RA; closed diamonds, FIGURE 8B), 13'RA-NR-AN-01 (NRAN01-13'RA; closed squares, FIGURE 8B). The experiments were conducted (and the results plotted) in a manner similar to those described above in regard to FIGURE 8A.

FIGURE 8C graphically depicts the results of *in vitro* studies investigating the effects on HT29 marker-negative cells of a 5 minute exposure to different concentrations of Roridin A ( open squares, FIGURE 8C), 2'RA-NR-AN-01 (NRAN01-2'RA; closed diamonds, FIGURE 8C), 13'RA-NR-AN-01 (NRAN01-13'RA; closed squares, FIGURE 8C). The experiments were conducted (and the results plotted) in a manner similar to those described above in regard to FIGURE 8A.

The results presented in FIGURES 8A-8C show slight differences between the different RA-NR-AN-01 conjugates at the highest doses, but at the lower doses the 2' and 13'RA-NR-AN-01 did not significantly inhibit target cell (i.e.,BO54 and A375) or non-target cell (i.e., HT29) metabolic activity over the long-term (i.e., 48 hours). Thus, the results suggest that the short-term inhibition of target cell protein synthesis (FIGURES 7C-7D, above) does not result in long-term metabolic effects on the cells, as measurable in MTT assays. That these assays were able to detect metabolic alterations in cells resulting from a 5 minute exposure is evidenced by the results obtained with free Roridin A. In this case, free Roridin A was non-specifically inhibitory to target and non-target cell types, even when the cells were exposed to the agent for only 5 minutes and then returned to culture for the 48-hour recovery period (FIGURES 8A-8C).

Thus, the findings with free Roridin A suggest that the MTT assay was capable of detecting metabolic alterations induced during a 5-minute exposure. Taken together these finding suggest that RA-NR-AN-01 conjugates can specifically inhibit target cell activity (i.e., protein synthesis) when administered in a "pulse" treatment, and that these effects were reversible without significant long-term effects on either protein synthesis or cellular metabolic activity (as measured in an MTT assay). These *in vitro* properties of the RA-NR-AN-01 conjugates were judged to be highly useful for inhibition of smooth muscle cell activity *in vivo*. Therefore, animal model studies were next conducted to evaluate the effects of these therapeutic conjugates *in vivo.*

### EXAMPLE 6

### Determination of Infusion Conditions in an Animal Model

The therapeutic conjugates of the invention are useful for inhibiting stenosis following vascular trauma or disease. In an illustrative example, vascular trauma that is induced during angioplasty is treated during the surgical procedure by removing the catheter used to perform the angioplasty, and inserting a balloon infusion catheter into the vessel. The infusion catheter is positioned with the instillation port, (or, alternatively, a permeable membrane region), in the traumatized area of the vessel, and then pressure is applied to introduce the therapeutic conjugate. For example, an infusion catheter with two balloons may be used and when one balloon is inflated on either side of the trauma site a fluid space is created that can be filled with a suitable infusion fluid containing the therapeutic conjugate. It has been reported previously infusion of a horseradish peroxidase (HRP) marker enzyme at a pressure of 300mm Hg over 45 seconds in dog or human coronary arteries resulted in penetration of the HRP into the vessel wall (6). However, HRP is a smaller molecule than NR-AN-01 and human and dog coronary arteries are also considerably smaller than the carotid or femoral arteries in the present domestic pig model system. Experiments were therefore conducted to determine, in a domestic pig model system, the infusion conditions suitable for delivery of a therapeutic conjugate to the vascular smooth muscle cells in carotid and femoral arterieis. Delivery conditions were monitored by evaluating the pentration of the therapeutic conjugate into the vascular wall, and specific binding of the therapeutic conjugate to the vascular smooth muscle cells in the vessel wall.

Using an infusion catheter the coronary and femoral arteries of domestic pigs or non-human primates were infused with NR-AN-01 for 45 seconds to 3 minutes at multiple pressures in the range of about 0.4 atmospheres (300mm Hg) to 3 atmospheres. After infusion the vessels were flushed with sterile saline and prepared for immunohistochemistry using HRP-conjugated goat anti-mouse IgG to detect the NR-AN-01 mouse IgG in the vessel wall. It was determined that full penetration was achieved of NR-AN-01 into these vessel walls at a pressure of 3 atmospheres after 3 minutes.

Immunohistology was also used to determine which animal model systems expressed the target antigen for NR-AN-01. Vascular tissue sections from redily available experimental animal species were exposed to NR-AN-01, washed, and reacted with HRP-conjugated goat anti-mouse IgG. Only non-human primates and swine were found to share the 250kD NR-AN-01 target antigen with man.

To determine whether NR-AN-01 could bind in a specific manner to its target antigen *in vivo*, the coronary and femoral arteries of domestic pigs were infused with therapeutic conjugates using an infusion catheter, the infusion sites were flushed with sterile saline, the surgical sites were then closed, and the animals were maintained for an additional 3-5 days. At the end of this time the vascular infusion sites were excised and prepared for immunohistology, once again using goat anti-mouse IgG to identify NR-AN-01. NR-AN-01 was identified in the vessel wall of swine coronary and femoral arteries 3-5 days after surgery, and the NR-AN-01 appeared to be associated only with vascular smooth muscle cells. These findings suggest that NR-AN-01 is capable of specifically binding to its target antigen in *vivo .*

### EXAMPLE 7

### Inhibition of Vascular Smooth Muscle Cells In Vivo

Intimal smooth muscle proliferation that follows balloon catheter-induced trauma is a good model to evaluate the efficacy therapeutic of conjugates for inhibiting smooth muscle cell activity *in vivo* in response to vascular trauma, including restenosis following angioplasty. Domestic pigs were used to study the effects of the NR-AN-01 (i.e., termed vascular smooth muscle binding protein or simply VSMBP in these studies; and therapeutic conjugates with Roridin A are termed VSMBP - RA). The events which normally follow balloon angioplasty in the porcine artery have been described previously (12). In these studies, dilation of the carotid artery using an oversized balloon (balloon: artery ratio approximately 1.5:1) resulted in complete endothelial denudation over an area of 1.5-2 cm in length. Although this length of traumatic injury was selected in an attempt to minimize thrombosis, there was still marked platelet deposition and thrombus formation. The procedure also resulted in dissection through the internal elastic lamina into the arterial media and necrosis of medial smooth muscle cells. Intimal thickening due to smooth muscle proliferation was apparent 7 days after injury and reached a mean maximum thickness of 85mm at 14 days. The histological appearance of this neointima is very similar to the proliferative neointimal tissue of human restenosis (13).

A single dose test protocol was conducted in domestic pigs with NR-AN-01-Roridin A conjugates. Localized administration of the test conjugates, i.e., through a catheter into a region of traumatized vessel confined by temporary slip ligatures, was designed to reduce systemic toxicity while providing a high level of exposure for the target smooth muscle cells. This intra-artery route of administration in animal model studies simulates the proposed route in human coronary arteries. The test protocol was designed as an initial *in vivo* screening of intra-arteriolar, site specific, catheter administered, vascular smooth muscle binding protein (VSMBP) conjugates. Toxicity of free drug was also evaluated, i.e., for pathobiological effects on arteriolar smooth muscle cells. The therapeutically effective dosage of the Roridin A-NR-AN-01 conjugate, was determined by *in vitro* studies and the proper intra-arteriolar administration pressure, was determined by administering free MAb and MAb conjugates to animals, as described above in Example 7.

Six domestic crossbred swine (Duroc X), weanling feeder pigs of approximately 30 pounds body weight, were used in the experiment. The animals were randomly assigned to the following treatment regime where each pig has four different treatments divided between the right and left carotid and femoral arteries, one of which is a PBS control (Tables 1-3, below).

**Table 1.**

| GROUP NO. | | |
|---|---|---|
| | TREATMENT GROUP | MATERIAL DESCRIPTION |
| 1 | CONTROL, VSMBP | VSMBP 200 mg/ml in PBS, pH 6.5 |
| | | |
| 2 | CONTROL, PBS | PBS, pH 6.5 in injection sterile water |
| | | |
| 3 | CONTROL, DRUG | Roridin A, 2.5 mg/ml in PBS, pH 6.5 |
| | | |
| 4 | TEST, CONJUGATE | VSMBP-RA2' (200 mg/ml VSHBP &2.5 mg/ml RA) |
| | | |
| 5 | TEST, CONJUGATE | VSMBP-RA13' (200 mg/ml VSMBP &3.1 mg/ml RA) |
| | | |
| 6 | TEST, CONJ+RA | VSMBP-RA2' (200ug/ml VSMBP &2.5 mg/ml RA) PLUS free Roridin A 2.5 mg/ml |
| | | |
| 7 | TEST, CONJ+RA | VSMBP-RA13' (200 mg VSMBP & 3.1ug/ml RA) PLUS free Roridin A 2.5 mg/ml |

### Surgical Procedure:

Test conjugates and control compounds were administered as a single intra-artery infusion at the site of endothelial denuding and trauma induced by a balloon catheter. Both the carotid and femoral arteries were abraded over 1 cm to 2 cm of endothelium by intraluminal passage of a 23 cm, size 3 (femoral) and size 4 (carotid), Uresil, Vascu-Flo, silicone occlusion balloon catheter, sufficiently distended with saline to generate slight resistance. This technique produced slight distension of the artery. Following this treatment, proximal and distal slip ligatures, 3-0 silk, were placed near the ends of the abraded region and a size 8 French, Infant Feeding Catheter (Cutter-Resiflex) attached to an Inflation Pro pressure syringe was used to administer the test conjugates and control compounds directly to the denuded segment at a pressure of three atmospheres for three minutes. The slip ligatures were removed after the three minute exposure period and arterial blood flow was re-established. In these studies, branches of the femoral or carotid arteries were ligated with 00 silk suture as required to attain pressurized infusion in the treated region. The largest distal branch of the femoral artery was incised and used as an entry site for the catheters which were then passed into the main femoral artery. Following this catheterization procedure in the main femoral artery the secondary branch was ligated. In these cases, ligation or incision was used to allow entry of the catheters and the opening was then closed with 3 to 4 sutures of 5-0 monosilamen polybutester (Novafil, D & G Monofil Inc., Monati, P.R. 00701.)

### Follow-up Procedures:

Following surgery the pigs were kept in 3 X 5 foot indoor runs with cement floors during the quarantine and surgical recovery periods. They were then transferred to indoor/outdoor pens for the remainder of the five week healing period prior to collection of tissues for histology.

The animals recovered normally from surgery with no evidence of hemorrhage or inflammation at the surgical sites. All six animals were examined 5 to 6 days after treatment with a doppler stethoscope and all arteries in each of the animals were patent. Post treatment all animals had normal appetite, activity and weight gain.

### Gross Pathology and Histological Evaluation:

Five weeks following the traumatization and treatment of the arteries the animals were sedated with Telazol (tiletamine hydrochloride) by intramuscular injection, heparinized (i.v. 2 ml sodium heparin 1000 units/ml), and euthanized by i.v. pentobarbitol. Both the right and left carotid and femoral arteries were removed with normal vessel included both proximal and distal to the treated segment. The arteries were measured and the location of ligatures and gross abnormalities noted. The arteries were transected at 2 mm intervals and arranged in order in cryomolds with O.C.T. compound (Tissue Tek, Miles Laboratories Inc., Elkhart, IN.46515.) and frozen in liquid nitrogen. The blocks were sectioned at 5 microns and stained with H&E, Massons Trichrome and Movats Pentachrome for morphological studies. Sections were also used for immunohistological staining of vascular smooth muscle.

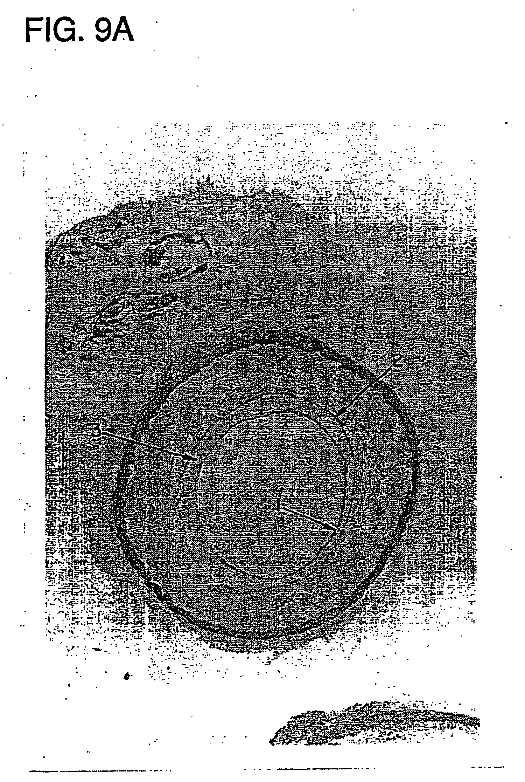

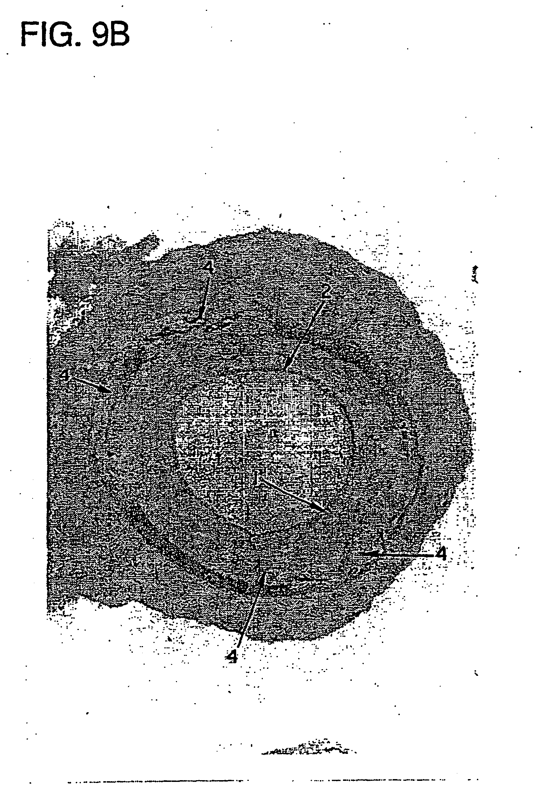

Histological examination of the step sections of the arteries revealed marked inhibition of intimal smooth muscle proliferation in the regions traumatized and treated with RA-NR-AN-01 conjugates (Table 2). This inhibition was evident even at sub-gross evaluation of the vessels. The inhibition of intimal smooth muscle cell proliferation was produced with minimal or no histological evidence of smooth muscle cell death in the artery wall. A cross-sections of one such traumatized artery is provided in FIGURES 9A and 9B.

**Table 2.**

| INTIMAL SMOOTH MUSCLE PROLIFERATION IN TRAUMATIZED AND TREATED PORCINE ARTERIES | | |
|---|---|---|
| TREATMENT | NO. ARTERIES EVALUATED | INTIMAL SMC HYPERTROPHY* |
| | | ave. (range) |
| Control, MAB | 4 | 3.75 (3-4) |
| Control, PBS | 4 | 4 (4) |
| Control, RA | 2 | 4 (4) |

| Test, 2'RA | | |
|---|---|---|
| (High pressure) | 1 | 1 (1) |
| (Low pressure) | 1 | 3 (3) |

| Test, 13'RA | | |
|---|---|---|
| (High pressure) | 1 | 1 (1) |
| (Low pressure) | 1 | 1(1) |

| | | |
|---|---|---|
| *Intimal SMC Hypertrophy, intimal smooth muscle cell hypertrophy scored on a scale from 1+ (minimal) to 4+ (maximal). | | |

The results presented in FIGURE 9A show (at 160x magnification) a cross-sectional of an untreated artery 5 weeks after angioplasty. Dominant histological features of the artery include displacement of the endothelium (see #1 in FIGURE 9A) away from the internal elastic lamina (see #2, FIGURE 9A) apparently due to intimal smooth muscle proliferation (see #3, FIGURE 9A).

The results presented in FIGURE 9B show (at 160x magnification) a cross-section of a treated artery 5 weeks after angioplasty and infusion of the RA-NR-AN-01 therapeutic conjugate. The vessel in this section was subjected to greater mechanical stresses than the vessel shown in FIGURE 9A, with multiple sites where the external elastic membrane was ruptured and associated proliferation of smooth muscle cells in the outer layers of the media (i.e., see #4 in FIGURE 9B). Treatment with therapeutic conjugate inhibited intimal hypertrophy, as evidenced by the lack of displacement of the endothelium (see #1, FIGURE 9B) from the internal elastic lamina (see #2, FIGURE 9B). Surprisingly, this inhibitory effect on intimal smooth muscle cells was accomplished without inhibiting hypertrophy of medial smooth muscle cells in the areas where the external elastic membrane was ruptured (see #4, FIGURE 9B).

This is a highly fortunate result because wound healing proceeds in the treated vessel without the adverse consequences of intimal hyperplasia and stenosis, or necrosis of smooth muscle cells in the media.

In these histological studies, comparisons were also made of the effectiveness of both the 2' and the 13' - Roridin A conjugate with the finding that the 13' conjugate (i.e., 13'RA-HS-NR-AH-01) appeared to be more active in inhibiting intimal hyperplasia of smooth muscle cells than the 2' conjugate (i.e., 2' RA-HS-NR-AN-01). In this study, low pressure infusion of the 13' conjugate appeared to inhibit smooth muscle proliferation more effectively than high pressure and the 13' conjugate also appeared to be more effective than the 2' conjugate.

In FIGURE 9B therapeutic conjugate administered at the site following angioplasty resulted in approximately 95% inhibition of the smooth muscle hypertrophy that restricted the lumen of the untreated vessel (FIGURE 9A). Significantly, the therapeutic conjugate exerted its effects on the smooth muscle cells migrating from the medial smooth muscle layers into the intima, without affecting either endothelium, or producing any signs of necrosis (i.e., cell death) in the smooth muscle cells in the medial layers of the arterial wall. Studies also failed to show any histological signs of mononuclear infiltration or fibrosis such as might result from toxic effects on the vessel wall. Also, visible signs of healing were observed in the intimal layers of treated vessels and with re-growth of endothelium observed i.e., endothelial cells growing over the thin layer of smooth muscle cells in the intima that lie between the endothelium and internal elastic lamina (i.e., #1 and #2, FIGURE 9B). These combined histological observations suggest the highly desirable features of wound healing, re-growth of endothelium and improved vascular strength following treatment with a therapeutic conjugate that inhibits smooth muscle hyperplasia in the intimal layers of the vessel.

### EXAMPLE 8

The ability of various therapeutic agents to inhibit DNA synthesis and protein synthesis in vascular smooth muscle cells was tested. ³H-leucine and ³H-thymidine uptake and cytotoxicity assays were conducted in accordance with the following protocols.

3H-leucine uptake: Vascular smooth muscle cells at 40,000 cells/ml were seeded in sterile 24 well plates at 1ml/well. The plates were incubated overnight at 37°C, 5% CO₂, 95% air in a humidified atmosphere (saturation). Log dilutions of the therapeutic agent of interest were incubated with the vascular smooth muscle cells for 5 minutes or 24 hours. Samples of the therapeutic agents were diluted in DMEM: F-12 media (Whittaker Bioproducts, Walkersville, Maryland) with 5% fetal bovine serum (GBS. Gibco) and 5% Serum Plus^{®} (JRH Biologicals). Following therapeutic agent incubation, the solution was aspirated, and 1ml/well of 0.5 microcurie/ml ³H-leucine in leucine free DMEM (Dulbecco's Modified Eagle's Medium) with 5% Serum Plus^{®} was added. The plates were re-incubated overnight at 37°C, 5% CO₂ in a humidified atmosphere. The cells were visually graded using an inverted microscope using a scoring scale to determine viability and cell number. The 1 to 3 grade is based upon percent of cell viability and number compared to control wells, with 3=100%, 2=70%-100% and 1=0%-70%. A record of this scoring assisted in determining the immediate cytotoxic effect of the therapeutic agents. The media was then aspirated, and the cells were washed twice with cold 5% TCA. 400 microliters of 0.2M NaOH was added per well, and the plates were incubated for two hours at room temperature on a rotating platform. 200 microliters per well of the cell solution was transferred into plastic scintillation vials (Bio-Rad Laboratories), and 4 milliliters of Bio-Safe^{®} II liquid scintillation fluid was added prior to vortexing. Vials were counted on a Beckman LS2800 Liquid Scintillation Counter interfaced with Beckman "Data Capture" software for conversion to a Lotus 1-2-3^{®} file and analysis using Lotus 1-2-3^{®}.

³H-thymidine uptake: Vascular smooth muscle cells were incubated in complete media with 5% FBS (Gibco) overnight at 37°C in a humidified, 5% CO₂ environment in sterile 24 well plates. The media was aspirated from the wells and serum free media supplemented with growth factors (DMEM: F-12 basal media supplemented with growth factor cocktail catalog number I1884 which contains insulin (5 micrograms/ml), transferrin (5 micrograms/ml) and sodium selenite (5 nanograms/ml) available from Sigma Chemical, St. Louis, Missouri) was added. Cells were incubated in this media for 24 hours. For a 5 minute therapeutic agent exposure, log dilutions of the therapeutic agent were incubated with the cells in complete media. After 5 minutes and media aspiration, 1 ml/well of 1.0 microcurie/ml ³H-thymidine dispersed in complete media was added. The 24 hour exposure involved incubation of the cells with 1 ml/well of 1.0 microcurie/ml of ³H-thymidine dispersed in complete media and log dilutions of the therapeutic agent being tested. In both exposure trials, the cells were then incubated overnight at 37°C in a humidified, 5% CO₂ environment. The cells were visually scored for viability and cell number. Cells were washed and prepared for transfer into plastic scintillation vials as described for the ³H-leucine protocol. Vials were counted on a Beckman LS2800 Liquid Scintillation Counter interfaced with Beckman "Data Capture" software for conversion to a Lotus 1-2-3° file and analysis using Lotus 1-2-30^{®}.

These protocols are amenable to use with other target cell populations, especially adherent monolayer cell types.

Morphological Cytotoxicity Evaluation: Vascular smooth muscle cells were seeded at 4.0 x 10⁴ cells/ml media/well on a commercially prepared four well slide (Nunc, Inc., Naperville, Illinois). Enough slides were seeded to accommodate two exposure lengths (5 minutes and 24 hours) and prescribed increment evaluation points (24 hours to 128 hours). All slides were run in duplicate to reveal any assay anomalies. The therapeutic agent was diluted in the same media used in the ³H-leucine and ³H-thymidine assays. Each four well slide was concentration bracketed to one log greater concentration (well "B"), one log lower concentration (well "D") of the minimal effect concentration (well "C"). As a control for normal morphology, one well (well "A") was left untreated (media only). Incubation took place in a 37°C, 5% CO₂ humidified incubator. After each of the two (5 minutes and 24 hours) exposure points, the therapeutic agent media was aspirated from each well, including the untreated well. One milliliter of fresh media was then added to replace the aspirated media. Re-incubation followed until each of the incremented evaluation points were achieved. At those points, the media was aspirated and subsequently replaced with 1 ml of 10% neutral buffered formalin for one hour to allow for proper fixation. These fixed slides were stained by hematoxylin (nuclear) and eosin (cytoplasmic) for morphologic evaluation and grading.

Results: The results of the 24 hour ³H-leucine protein inhibition assay and the 24 hour ³H-thymidine DNA synthesis inhibition assay are shown in Figs. 10A-10C for suramin, staurosporin and nitroglycerin, respectively. All of the tested compounds showed an available therapeutic range (area under the curve of ³H-leucine assay is greater than that resulting from the ³H-thymidine assay) indicating usefulness in the practice of sustained release dosage form embodiments of the present invention. More specifically, the compounds inhibited the ability of vascular smooth muscle cells to undergo DNA synthesis in the presence of 5% FBS to a greater extent than they inhibited protein synthesis of vascular smooth muscle cells.

### EXAMPLE 9

The ability of vascular smooth muscle cells to bind and internalize particles coated with binding protein or peptide was demonstrated with monoclonal antibody (NR-AN-01) coated gold beads both in vitro and in vivo. The vascular smooth muscle cell tissue cultures (B054), an antigen positive control cell line (A375) and an antigen negative control cell line (HT29) were incubated with 10 nm gold beads, with one group coated with NR-AN-01 and a second, uncoated control group. The cells were exposed to the beads as monolayer and cell suspension cultures and were examined at six time points (i.e., 1 minute, 5 minutes, 15 minutes, 30 minutes, 60 minutes and 24 hours) for binding and internalization by electron microscopy.

Table 3 shows the results of the experimentation, indicating that the binding to the cell surface is specific. If aggregates of particles settled on the monolayer surface of both the smooth muscle cells and the control cells, the particles were nonspecifically internalized by macro and micro phagocytosis. When the cells were maintained in a cell suspension, non-specific internalization was minimal or absent. Non-specific adherence of gold beads devoid of NR-AN-01 to surface mucin produced by HT29 cells was observed, resulting in modest non-specific internalization thereof. Vascular smooth muscle cell uptake of NR-AN-01 targeted gold beads was highly specific in cell suspension cultures.

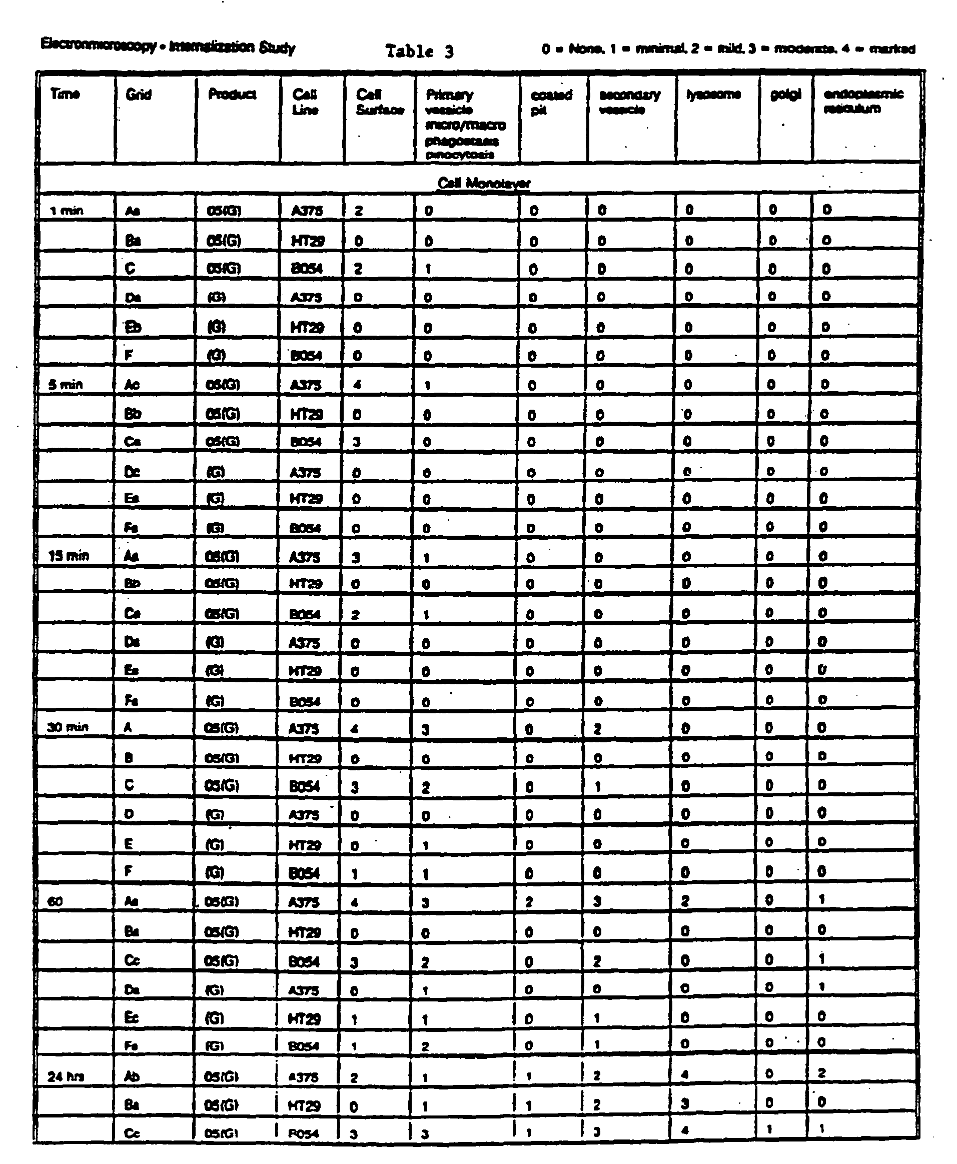

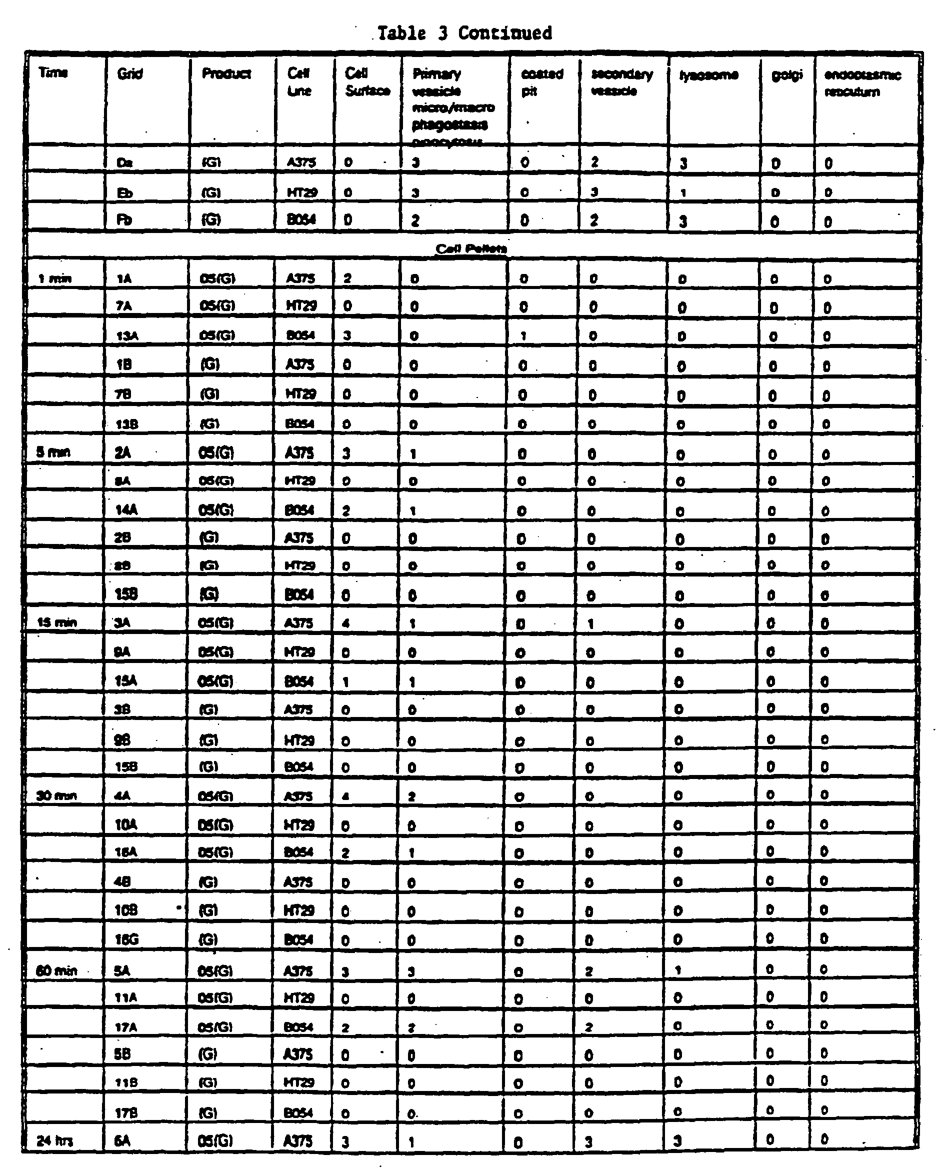

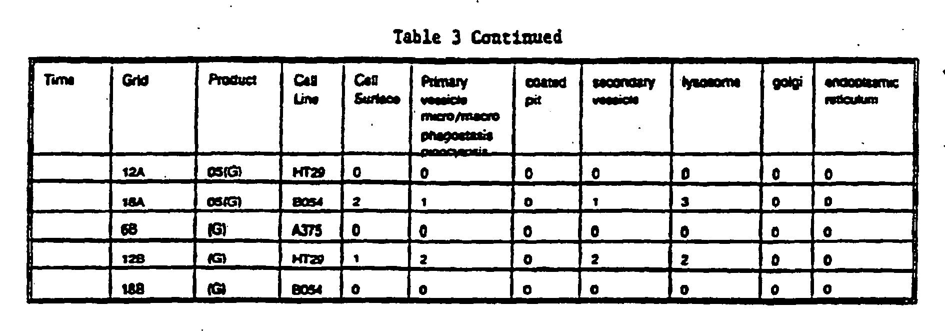

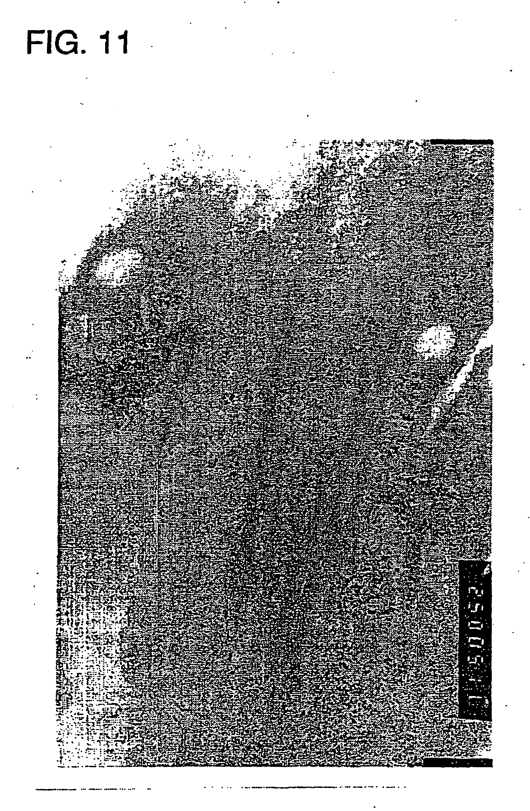

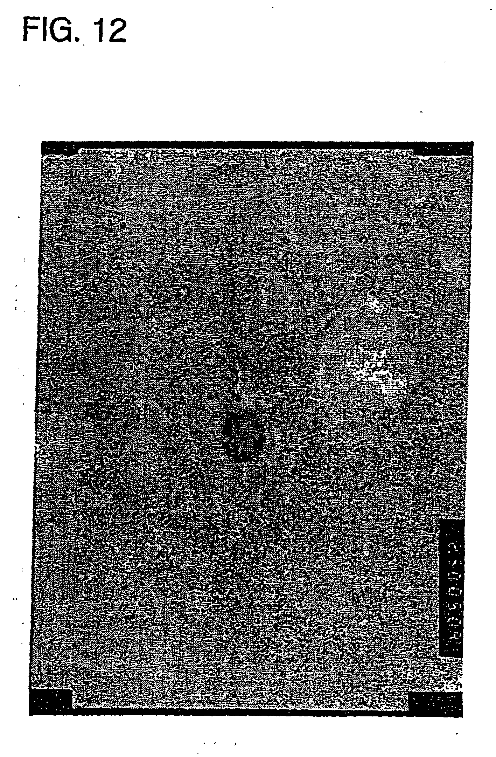

FIGURE 11 shows a tangential section parallel to the inner surface of a smooth muscle cell characterized by numerous endocytic vesicles, several of which contain antibody coated gold beads in the process of being internalized by the cell. These endocytic vesicles with particles attached to cell surface antigens were stimulated to fuse with lysosomes at a higher than expected rate for normal cell surface membrane recycling. The resultant marked accumulation of internalized particles was observed at the 24 hour time point and is shown in FIGURE 12.

The targeted gold bead vascular smooth muscle cell surface binding, internalization and lysosome concentration was observed in vivo as well. NR-AN-01 coated gold beads were infused via intravascular catheter, open ended with treated area occluded proximally and distally with slip ligatures, at 3 atm pressure applied for 3 minutes into the wall of a pig femoral artery immediately following balloon trauma. The bead internalization rate varied with the degree of damage sustained by the vascular smooth muscle cell during the balloon trauma. Cells with minimal or no damage rapidly internalized the particles by endocytosis and phagocytosis, concentrating the internalized particles in lysosomes. Cells that were killed by the trauma exhibited surface bead binding. Cells that were damaged by the trauma but survived were characterized by bead surface binding with delayed internalization and lysosome concentration. FIGURE 3 shows particulate concentration in the lysosomes in vivo at one week following bead administration.

### Citations

1. Popma, J.J. et al. 1990. Factors influencing restenosis after coronary angioplasty. Amer. J. Med. 88: 16N-24N.
2. Fanelli, C. et al. 1990. Restenosis following coronary angioplasty. Amer. Heart Jour. 119: 357-368.
3. Johnson, D.E. et al. 1988. Coronary atherectomy: Light microscopic and immunochemical study of excised tissue (abstract). Circulation 78 (Suppl. II): 11-82.
4. Liu, M.W. et al. 1989. Restenosis after coronary angioplasty; Potential biologic determinants and role of intimal hyperplasia. Circulation 79: 1374-1387.
5. Clowes, A.W. et al. 1985. Significance of quiescent smooth muscle migration in the injured rat carotic artery. Circ. Res. 56: 139-145.
6. Goldman, B. et al. 1987. Influence of pressure on permeability of normal and diseased muscular arteries to horseradish peroxidase; A new catheter approach. Atheroscleosis 65: 215-225.
7. Wolinsky, H. et al. 1990. Use of a perforated balloon catheter to deliver concentrated heparin into the wall of the normal canine artery. JACC 15 (2): 475-481.
8. Nabel, E.G. et al. 1989. Recombinant gene expression in vivo within endothelial cells of the arterial wall. Science 244: 1342-1344.
9. Middlebrook, J.L. et al. 1989. Binding of T-2 toxin to eukaryotic cell ribosomes. Biochem. Pharm. 38 (18): 3101-3110.
10. Barbacid, M. et al. 1974. Binding of [acetyl-¹⁴C] trichodermin to the peptidyl transferase center of eukaryotic ribosomes. Eur. J. Biochem. 44: 437-444.
11. Sclingemann et al. 1990. Am. J. Pathol. 136: 1393-1405.
12. Steele P.M., Chesebro J.H., Stanson A.W., et al. 1985. Balloon angioplasty: natural history of the pathophysiological response to injury in a pig model. Circ. Res. 57:105-112.
13. Schwartz, R.S., Murphy J.G., Edwards W.D., Camrud A.R., Vliestra R.E., Holmes D.R. Restenosis after balloon angioplasty. A practical proliferative model in porcine coronary arteries. Circulation 1990; 82:2190-2200.
14. Bumol, T.F. and R.A. Reisfeld. 1982. Unique glycoprotein-proteoglycan complex defined by monoclonal antibody on human melanoma cells. Proc. Natl. Acad. Sci. USA 79:1245-1249.

While the preferred embodiment of the invention has been illustrated and described, it will be appreciated that various changes can be made therein without departing from the spirit and scope of the invention.

## Claims

1. A therapeutic conjugate comprising a binding protein or peptide coupled to a therapeutic agent, wherein said binding protein or peptide specifically binds to the cell membranes of either vascular smooth muscle cells or pericytes, or which binds to the interstitial matrix of the artery wall, and wherein said therapeutic agent alters the cellular metabolism, or inhibits a cellular activity of the smooth muscle cells or pericytes.

2. The therapeutic conjugate of claim 1, wherein the inhibited cell activity is selected from at least one of protein synthesis, DNA synthesis, spindle fiber formation, cellular proliferation, cell migration, microtubule formation, microfilament formation, extracellular matrix synthesis, extracellular matrix secretion, or increase in cell volume.

3. The therapeutic conjugate of claim 1, wherein the therapeutic agent does not substantially kill the target cell.

4. The therapeutic conjugate of claim 1, wherein the therapeutic agent is cytotoxic.

5. The therapeutic conjugate of claim 4, wherein the cytotoxic therapeutic agent is a sesquiterpenoid mycotoxin.

6. The therapeutic conjugate of claim 5, wherein the sesquiterpenoid mycotoxin is verrucarin or roridin.

7. The therapeutic conjugate of claim 1, wherein the therapeutic agent is cytostatic.

8. The therapeutic conjugate of claim 7, wherein the cytostatic therapeutic agent is selected from at least one of modified toxins, methotrexate, adriamycin, radionuclides, peptidic or mimetic inhibitors, or protein kinase inhibitors.

9. The therapeutic conjugate of claim 7, wherein the cytostatic therapeutic agent is selected from at least one of subfragments of heparin, Triazolopryimidine, Lovastatin, or prostaglandins E1 or I2.

10. The therapeutic conjugate of claim 1, wherein the therapeutic agent is a cytoskeletal inhibitor.

11. The therapeutic conjugate of claim 10, wherein the cytoskeletal inhibitor is colchicines or vinblastin.

12. The therapeutic conjugate of claim 1, wherein the therapeutic agent is a metabolic inhibitor.

13. The therapeutic conjugate of claim 12, wherein the metabolic inhibitor is selected from at least one of triochothecenes, modified diphtheria, ricin toxins, or *Pseudomonas exotoxin.*

14. The therapeutic conjugate of claim 1, wherein the vascular smooth muscle cell binding protein or peptide is specifically associated with a chondroitin sulfate proteoglycan expressed on the membranes of the vascular smooth muscle cell.

15. The therapeutic conjugate of claim 14, wherein the chondroitin sulfate proteoglycan has a molecular weight of about 400 kD.

16. The therapeutic conjugate of claim 14, wherein the chondroitin sulfate proteoglycan has a molecular weight of about 250 kD.

17. The therapeutic conjugate of claim 14, wherein the vascular smooth muscle cell binding protein or peptide specifically associates with the chondroitin sulfate proteoglycan at an association constant of at least 10⁴ liter/mole.

18. The therapeutic conjugate of claim 1, wherein the binding protein or peptide is localized to intercellular stroma and matrix located between and among vascular smooth muscle cells.

19. The therapeutic conjugate of claim 18, wherein the binding protein or peptide is specifically associated with collagen, extracellular glycoproteins, tenascin, reticulum fibers, elastic fibers, or other intercellular matrix compounds.

20. The therapeutic conjugate of claim 1, wherein the tumor cell binding protein or peptide is associated with cell surface markers expressed by the target tumor cell or cytoplasmic epitopes thereof.

21. The therapeutic conjugate of claim 20, wherein the tumor cell binding protein or peptide is associated with epitopes of myc, ras, bcr/Ab1, erbB, mucins, II-6, EGF, or TGF.

22. The therapeutic conjugate of claim 1, wherein the immune system effector cell binding protein or peptide is associated with cell surface markers of the target immune system effector cell or cytoplasmic epitopes thereof.

23. The therapeutic conjugate of claim 22, wherein the immune system effector cell binding protein or peptide is anti-TAC or IL-2.

24. The therapeutic conjugate of any one of claims 1-23, wherein the Therapeutic agent is in a dosage form.

25. The therapeutic conjugate of claim 24, wherein the dosage form is biodegradable.

26. The therapeutic conjugate of claim 24, wherein the dosage form comprises microparticulates, nanoparticulates, or a mixture thereof.

27. The therapeutic conjugate of claim 26, wherein the microparticulates or nanoparticulates comprise a polymer derived from the condensation of alpha hydroxycarboxylic acids and related lactones.

28. The therapeutic conjugate of claim 26, wherein the microparticulates or nanoparticulates are formed of a mixture of thermoplastic polyesters.

29. The therapeutic conjugate of claim 28, wherein the thermoplastic polyester is polylactide, polyglycolide, or a copolymer of lactide and glycolide.

30. The therapeutic conjugate of claim 24, wherein the dosage form is a sustained release dosage form capable of releasing the therapeutic agent over a time period.

31. The therapeutic conjugate of claim 30, wherein the time period for release of the therapeutic agent ranges from about 3 to about 180 days.

32. The therapeutic conjugate of claim 31, wherein the time period for release of the therapeutic agent ranges from about 10 to about 21 days.

33. A use of the therapeutic conjugate of any one of claims 1-32 for the preparation of a therapeutically effective dosage of a medicament for the reduction of stenosis following angioplasty.

34. The use of claim 33, wherein said medicament is in a form suitable for administration with a catheter at a site of administration in a subject.

35. The use of claim 33, wherein said medicament is in a form suitable for administration with an infusion needle at a site of administration in a subject.

## Patentansprüche

1. Therapeutisches Konjugat umfassend ein bindendes Protein oder Peptid, verbunden mit einem therapeutischen Mittel, wobei das bindende Protein oder Peptid spezifisch an Zellmembranen entweder glatter Gefäßmuskelzellen oder Perizyten bindet, oder an die interstitielle Matrix der Arterienwand bindet, und wobei das therapeutische Mittel den zellulären Metabolismus verändert oder eine zelluläre Aktivität der glatten Gefäßmuskelzellen oder Perizyten inhibiert.

2. Das therapeutische Konjugat nach Anspruch 1, wobei die inhibierte Zellaktivität ausgewählt ist aus mindestens einer der Möglichkeiten Proteinsynthese, DNA-Synthese, Spindelfaserbildung, zelluläre Proliferation, Zellmigration, Mikrotubulibildung, Mikrofilamentbildung, Synthese extrazellulärer Matrix, Sekretion extrazellulärer Matrix, oder Steigerung des Zellvolumens.

3. Das therapeutische Konjugat nach Anspruch 1, wobei das therapeutische Mittel die Zielzelle im Wesentlichen nicht abtötet.

4. Das therapeutische Konjugat nach Anspruch 1, wobei das therapeutische Mittel zytotoxisch ist.

5. Das therapeutische Konjugat nach Anspruch 4, wobei das zytotoxische therapeutische Mittel ein Sesquiterpenoid-Mykotoxin ist.

6. Das therapeutische Konjugat nach Anspruch 5, wobei das Sesquiterpenoid-Mykotoxin Verrucarin oder Roridin ist.

7. Das therapeutische Konjugat nach Anspruch 1, wobei das therapeutische Mittel zytostatisch ist.

8. Das therapeutische Konjugat nach Anspruch 7, wobei das zytostatische therapeutische Mittel ausgewählt ist aus mindestens einer der Möglichkeiten Modifizierte Toxine, Methotrexat, Adriamycin, Radionukliden, peptidischen oder mimetischen Inhibitoren, oder Proteinkinaseinhibitoren.

9. Das therapeutische Konjugat nach Anspruch 7, wobei das zytostatische therapeutische Mittel ausgewählt ist aus mindestens einer der Möglichkeiten Subfragmente von Heparin, Triazolpyrimidin, Lovastatin, oder Prostaglandinen E1 oder 12.

10. Das therapeutische Konjugat nach Anspruch 1, wobei das therapeutische Mittel ein zytoskelettaler Inhibitor ist.

11. Das therapeutische Konjugat nach Anspruch 10, wobei der zytoskelettale Inhibitor Colchicin oder Vinblastin ist.

12. Das therapeutische Konjugat nach Anspruch 1, wobei das therapeutische Mittel ein metabolischer Inhibitor ist.

13. Das therapeutische Konjugat nach Anspruch 12, wobei der metabolische Inhibitor ausgewählt ist aus mindestens einer der Möglichkeiten Trichothecenen, modifizierten Diphterie-, Ricin-Toxinen oder Pseudomonas-Exotoxin.

14. Das therapeutische Konjugat nach Anspruch 1, wobei das an glatte Gefäßmuskelzellen bindende Protein oder Peptid spezifisch mit einem Chondroitinsulfat-Proteoglykan verknüpft ist, das auf den Membranen der glatten Gefäßmuskelzelle exprimiert wird.

15. Das therapeutische Konjugat nach Anspruch 14, wobei das Chondroitinsulfat-Proteoglykan ein Molekulargewicht von etwa 400 kD aufweist.

16. Das therapeutische Konjugat nach Anspruch 14, wobei das Chondroitinsulfat-Proteoglykan ein Molekulargewicht von etwa 250 kD aufweist.

17. Das therapeutische Konjugat nach Anspruch 14, wobei das die glatte Gefäßmuskelzelle bindende Protein oder Peptid spezifisch mit einer Assoziationskonstante von 10⁴l/Mol an das Chondroitinsulfat-Proteoglykan bindet.

18. Das therapeutische Konjugat nach Anspruch 1, wobei das bindende Protein oder Peptid an intrazelluläres Stroma und Matrix zwischen glatten Muskelzellen und um diese herum lokalisiert ist.

19. Das therapeutische Konjugat nach Anspruch 18, wobei das bindende Protein oder Peptid spezifisch an Kollagen, extrazellulären Glykoproteinen, Tenascin, retikulären Fasern, elastischen Fasern oder anderen Verbindungen der interzellulären Matrix gebunden wird.

20. Das therapeutische Konjugat nach Anspruch 1, wobei das Tumorzellen-bindende Protein oder Peptid an Zelloberflächenmarker, oder deren cytoplasmatische Epitope, gebunden wird, die von der Zieltumorzelle exprimiert werden.

21. Das therapeutische Konjugat nach Anspruch 20, wobei das Tumorzellen-bindende Protein oder Peptid an Epitope von myc, ras, bcr/Ab1, erbB, Mucinen, IL-6, EGF oder TGF gebunden wird.

22. Das therapeutische Konjugat nach Anspruch 1, wobei das Immunsystemeffektorzellen-bindende Protein oder Peptid an Zelloberflächenmarker der Zielimmunsystemeffektorzelle oder deren cytoplasmatische Epitope gebunden wird.

23. Das therapeutische Konjugat nach Anspruch 22, wobei das Immunsystemeffektorzellen-bindende Protein oder Peptid anti-TAC oder IL-2 ist.

24. Das therapeutische Konjugat nach einem der Ansprüche 1-23, wobei das therapeutische Mittel in einer Dosierungsform vorliegt.

25. Das therapeutische Konjugat nach Anspruch 24, wobei die Dosierungsform bioabbaubar ist.

26. Das therapeutische Konjugat nach Anspruch 24, wobei die Dosierungsform Mikropartikel, Nanopartikel oder ein Gemisch aus diesen umfasst.

27. Das therapeutische Konjugat nach Anspruch 26, wobei die Mikropartikel oder Nanopartikel ein Polymer umfassen, das aus der Kondensation von Hydroxycarbonsäuren und verwandten Laktonen abgeleitet ist.

28. Das therapeutische Konjugat nach Anspruch 26, wobei die Mikropartikel oder Nanopartikel aus einem Gemisch thermoplastischer Polyester gebildet ist.

29. Das therapeutische Konjugat nach Anspruch 26, wobei der thermoplastische Polyester ein Polylactid, ein Polyglycolid, oder ein Copolymer aus Lactid und Glycolid ist.

30. Das therapeutische Konjugat nach Anspruch 24, wobei die Dosierungsform eine Retarddosierungsform ist, welche in der Lage ist, das therapeutische Mittel über eine Zeitspanne freizusetzen.

31. Das therapeutische Konjugat nach Anspruch 30, wobei die Zeitspanne zur Freisetzung des therapeutischen Mittels im Bereich von etwa 3 bis etwa 180 Tage liegt.

32. Das therapeutische Konjugat nach Anspruch 31, wobei die Zeitspanne zur Freisetzung des therapeutischen Mittels im Bereich von etwa 10 bis etwa 21 Tage liegt.

33. Verwendung des therapeutischen Konjugats nach einem der Ansprüche 1-32 zur Herstellung einer therapeutisch wirksamen Dosierung eines Arzneimittels zur Verminderung von Stenose nach Angioplastie.

34. Die Verwendung nach Anspruch 33, wobei das Arzneimittel in einer Form vorliegt, die zur Verabreichung mit einem Katheter an einem Verabreichungsort in einem Patienten geeignet ist.

35. Die Verwendung nach Anspruch 33, wobei das Arzneimittel in einer Form vorliegt, die zur Verabreichung mit einer Infusionsnadel an einem Verabreichungsort in einem Patienten geeignet ist.

## Revendications

1. Conjugué thérapeutique comprenant une protéine ou un peptide de liaison couplée à un agent thérapeutique, dans lequel ladite protéine ou ledit peptide de liaison est spécifiquement liée aux membranes cellulaires de cellules musculaires lisses vasculaires ou de péricytes, ou qui est liée à la matrice interstitielle de la paroi artérielle, et dans lequel ledit agent thérapeutique modifie le métabolisme cellulaire, ou inhibe une activité cellulaire des cellules musculaires lisses ou des péricytes.

2. Conjugué thérapeutique selon la revendication 1, dans lequel l'activité cellulaire inhibée est sélectionnée à partir d'au moins une des synthèse protéinique, synthèse d'ADN, formation de fibre fusiforme, prolifération cellulaire, migration cellulaire, formation de microtubule, formation de microfilament, synthèse de matrice extracellulaire, sécrétion de matrice extracellulaire, ou augmentation de volume cellulaire.

3. Conjugué thérapeutique selon la revendication 1, dans lequel l'agent thérapeutique ne tue pas substantiellement la cellule cible.

4. Conjugué thérapeutique selon la revendication 1, dans lequel l'agent thérapeutique est cytotoxique.

5. Conjugué thérapeutique selon la revendication 4, dans lequel l'agent thérapeutique cytotoxique est une mycotoxine sesquiterpenoïde.

6. Conjugué thérapeutique selon la revendication 5, dans lequel la mycotoxine sesquiterpenoïde est verrucarine ou roridine.

7. Conjugué thérapeutique selon la revendication 1, dans lequel l'agent thérapeutique est cytostatique.

8. Conjugué thérapeutique selon la revendication 7, dans lequel l'agent thérapeutique cytostatique est sélectionné à partir d'au moins une des toxines modifiées, methotrexate, adriamycine, radionucléides, inhibiteurs peptidiques ou mimétiques, ou inhibiteurs de protéine kinase.

9. Conjugué thérapeutique selon la revendication 7, dans lequel l'agent thérapeutique cytostatique est sélectionné à partir d'au moins un des sous-fragments d'héparine, de Triazolopyrimidine, de Lovastatine, ou de prostaglandines E1 ou I2.

10. Conjugué thérapeutique selon la revendication 1, dans lequel l'agent thérapeutique est un inhibiteur cytosquelettique.

11. Conjugué thérapeutique selon la revendication 10, dans lequel l'inhibiteur cytosquelettique est composé de colchicines ou de vinblastine.

12. Conjugué thérapeutique selon la revendication 1, dans lequel l'agent thérapeutique est un inhibiteur métabolique.

13. Conjugué thérapeutique selon la revendication 12, dans lequel l'inhibiteur métabolique est sélectionné à partir d'au moins un des trichotécènes, diphtérie modifiée, toxines de ricin, ou exotoxine de Pseudomonas.

14. Conjugué thérapeutique selon la revendication 1, dans lequel le peptide ou la protéine de liaison cellulaire de muscle lisse vasculaire est associée de façon spécifique à un protéoglycane de chondroitine sulfate exprimé sur les membranes de la cellule de muscle lisse vasculaire.

15. Conjugué thérapeutique selon la revendication 14, dans lequel le protéoglycane de chondroitine sulfate a une masse moléculaire d'environ 400 kD.

16. Conjugué thérapeutique selon la revendication 14, dans lequel le protéoglycane de chondroitine sulfate a une masse moléculaire d'environ 250 kD.

17. Conjugué thérapeutique selon la revendication 14, dans lequel le peptide ou la protéine de liaison cellulaire de muscle lisse vasculaire s'associe de manière spécifique au protéoglycane de chondroitine sulfate à une constante d'association d'au moins 10⁴ litre/mole.

18. Conjugué thérapeutique selon la revendication 1, dans lequel le peptide ou la protéine de liaison se trouve dans la matrice et dans le stroma intercellulaire situés entre et parmi des cellules de muscles lisses vasculaires.

19. Conjugué thérapeutique selon la revendication 18, dans lequel le peptide ou la protéine de liaison est associée de manière spécifique à du collagène, des glycoprotéines extracellulaires, de la ténascine, des fibres de réticulum, des fibres élastiques, ou d'autres composés de matrice intercellulaire.

20. Conjugué thérapeutique selon la revendication 1, dans lequel le peptide ou la protéine de liaison cellulaire tumorale est associée à des marqueurs de surface cellulaire exprimés par la cellule tumorale cible ou des épitopes cytoplasmiques de celle-ci.

21. Conjugué thérapeutique selon la revendication 20, dans lequel le peptide ou la protéine de liaison cellulaire tumorale est associée à des épitopes de myc, ras, bcr/Ab1, erbB, mucines, II-6, EGF, ou TGF.

22. Conjugué thérapeutique selon la revendication 1, dans lequel le peptide ou la protéine de liaison de cellule effectrice du système immunitaire est associée à des marqueurs de surface cellulaire de cellule effectrice du système immunitaire cible ou à des épitopes cytoplasmique de celle-ci.

23. Conjugué thérapeutique selon la revendication 22, dans lequel la protéine ou le peptide de liaison de cellule effectrice du système immunitaire est anti-TAC ou IL-2.

24. Conjugué thérapeutique selon l'une quelconque des revendications 1 à 23, dans lequel l'agent thérapeutique est sous forme posologique.

25. Conjugué thérapeutique selon la revendication 24, dans lequel la forme posologique est biodégradable.

26. Conjugué thérapeutique selon la revendication 24, dans lequel la forme posologique comprend des microparticules, des nanoparticules, ou un mélange de celles-ci.

27. Conjugué thérapeutique selon la revendication 26, dans lequel les microparticules ou les nanoparticules comprennent un polymère dérivé de la condensation d'acides hydroxycarboxyliques alpha et de lactones apparentés.

28. Conjugué thérapeutique selon la revendication 26, dans lequel les microparticules ou les nanoparticules sont formées d'un mélange de polyesters thermoplastiques.

29. Conjugué thérapeutique selon la revendication 28, dans lequel le polyester thermoplastique est du polylactide, du polyglycolide, ou un copolymère de lactide et de glycolide.

30. Conjugué thérapeutique selon la revendication 24, dans lequel la forme posologique est une forme posologique à libération lente capable de libérer l'agent thérapeutique sur un intervalle de temps.

31. Conjugué thérapeutique selon la revendication 30, dans lequel l'intervalle de temps de libération de l'agent thérapeutique s'étend d'environ 3 à environ 180 jours.

32. Conjugué thérapeutique selon la revendication 31, dans lequel l'intervalle de temps de libération de l'agent thérapeutique s'étend d'environ 10 à environ 21 jours.

33. Utilisation du conjugué thérapeutique selon l'une quelconque des revendications 1 à 32, pour la préparation d'une dose thérapeutiquement efficace d'un médicament pour la réduction d'une sténose consécutive à une angioplastie.

34. Utilisation selon la revendication 33, dans laquelle ledit médicament est sous une forme adaptée pour une administration par cathéter à un site d'administration chez un sujet.

35. Utilisation selon la revendication 33, dans laquelle ledit médicament est sous une forme adaptée pour une administration par aiguille de perfusion à un site d'administration chez un sujet.
